# EUROPEAN PATENT APPLICATION

(11) **EP 2 213 751 A1**
(43) Date of publication of application: **04.08.2010**
(21) Application number: 08844837.8
(22) Date of filing: 30.10.2008
(51) Int. Cl.: C12Q 1/68, G01N 33/543, C12N 15/09, G01N 33/53

(54) **POLYMER FOR DETECTION OF TARGET SUBSTANCE, AND METHOD FOR DETECTION OF TARGET SUBSTANCE**

(30) Priority: 31.10.2007 JP 2007283672; 12.06.2008 JP 2008153974
(71) Applicant: Eisai R&D Management Co., Ltd., Tokyo 112-8088 (JP)
(72) Inventor: KANASHIMA, Motohito, Kawasaki-shi Kanagawa 210-0855 (JP); HAKII, Chikako, Kawasaki-shi Kanagawa 210-0855 (JP); USUI, Mitsugu, Kawasaki-shi Kanagawa 210-0855 (JP); YAMADA, Takashi, Kawasaki-shi Kanagawa 210-0821 (JP); HONMA, Hiroaki, Kawasaki-shi Kanagawa 210-0855 (JP)
(74) Representative: Grünecker, Kinkeldey, Stockmair & Schwanhäusser Anwaltssozietät
(86) International application number: PCT/JP2008/069778
(87) International publication number: WO 2009/057702

(57) **Abstract**

Provided is a method of detecting a target substance by which the target substance can be detected with efficiency and a high sensitivity, and in a simple manner, a target substance detection polymer used in the method, and a method of forming the polymer. The method of detecting a target substance includes the steps of: (A) forming a target substance detection polymer by causing multiple kinds of nucleic acid probes for forming a polymer to react with a binding probe having a region capable of binding to the target substance and a region capable of binding to at least one of the nucleic acid probes in a solution; (B) binding the target substance detection polymer and the target substance; and (C) detecting the target substance detection polymer to which the target substance is bound.

## Description

### Technical Field

The present invention relates to a target substance detection polymer, a method of forming the polymer, and a method of detecting a target substance with the polymer.

### Background Art

Patent Documents 1 to 4 each disclose a method involving using multiple kinds of nucleic acid probes having base sequences complementary to each other to form an assembly (a polymer) of the nucleic acid probes by a probe alternation link self-assembly reaction (hereinafter, a method of forming a polymer on the basis of a probe alternation link self-assembly reaction is referred to as "a PALSAR method").

Patent Document 1 discloses, as a method of detecting a target gene involving the employment of the PALSAR method, a method involving causing a probe having a region complementary to nucleic acid probes used for polymer formation and a region complementary to a target nucleic acid to react with the target gene, adding the multiple nucleic acid probes for polymer formation after the reaction, forming a self assembly by a probe alternation link self-assembly reaction, amplifying a signal, and detecting the target gene.
In addition, Patent Document 1 discloses, as a method of detecting an antigen and an antibody involving the employment of the PALSAR method, a method involving binding the antibody to the antigen, then using a biotinylated gene having a region complementary to nucleic acid probes used for polymer formation, protein A, and streptavidin to form a complex of the antigen, the antibody, protein A, streptavidin, and the biotinylated gene, then adding the multiple nucleic acid probes for polymer formation, forming a self assembly by a probe alternation link self-assembly reaction, amplifying a signal, and detecting the antigen and the antibody.

In addition, Patent Document 5 discloses, as a method of detecting a target gene involving the employment of the PALSAR method, a method involving binding a capture probe capable of capturing a target nucleic acid to a reaction substrate such as a microplate to capture the target nucleic acid, then adding a binding probe having a region complementary to nucleic acid probes used for polymer formation and a region complementary to the target nucleic acid to form a complex of the capture probe, the target nucleic acid, and the binding probe, then adding the multiple nucleic acid probes for polymer formation, forming a self assembly by a probe alternation link self-assembly reaction, amplifying a signal, and detecting the target nucleic acid.

Although the above methods can significantly improve detection sensitivities for target substances such as a target gene, and an antigen and an antibody, each of the methods has involved the following problem. That is, the probe alternation link self-assembly reaction of nucleic acid probes is performed on the surface of a solid phase to which a target substance is bound, and hence a condition for a buffer and a temperature condition at the time of the reaction are restricted. In particular, it has not been easy to perform the probe alternation link self-assembly reaction efficiently because of the following reason. That is, the concentration of each of the nucleic acid probes used for polymer formation must be increased in order that a large polymer may be formed, but reaction conditions are limited in order that a large polymer may be formed on the solid phase within a short time period. Further, increasing the concentration of each of the nucleic acid probes involves such a problem that the amount of nucleic acid probes that do not contribute to the reaction increases and the frequency of physical adsorption to the solid phase or of a non-specific reaction increases in association with the increase. Accordingly, it is desired that the largest effect be exerted while the concentration of each of the nucleic acid probes is minimized.

In addition, when a protein or low-molecular weight substance is subjected to measurement by an antigen-antibody reaction, the reaction is typically performed at a temperature of 25 to 37°C because the antibody or the protein is poor in heat stability. However, a probe alternation link self-assembly reaction based on the PALSAR method is performed in a temperature region of 45°C to 65°C where the antibody or the protein is denatured, and hence it has been difficult to efficiently perform the antigen-antibody reaction involving the employment of the PALSAR method.
Patent Document 1: JP 3267576 B
Patent Document 2: JP 3310662 B
Patent Document 3: WO 02/31192
Patent Document 4: JP 2002-355081 A
Patent Document 5: WO 03/029441

### Disclosure of the Invention

### Problems to be solved by the Invention

An object of the present invention is to provide a method of detecting a target substance by which the target substance can be detected with efficiency and a high sensitivity, and in a simple manner, a target substance detection polymer used in the method, and a method of forming the polymer.

### Means for solving the Problems

The inventors of the present invention have made extensive studies with a view to solving the above problems. As a result, the inventors have found that restraints on conditions such as a reaction solution can be alleviated, and further, a detection sensitivity can be significantly improved by causing nucleic acid probes for polymer formation to react with a binding probe to form a polymer capable of binding to a target substance and then causing the polymer to react with the target substance.
It should be noted that the term "binding probe" as used in the description refers to a probe having a portion capable of directly or indirectly binding to the target substance to be detected, and having a region capable of binding to at least one of the multiple nucleic acid probes used for polymer formation.

A first aspect of the method of detecting a target substance of the present invention comprises the steps of: (A) forming a target substance detection polymer by causing multiple kinds of nucleic acid probes for forming a polymer to react with a binding probe comprising a target region capable of directly or indirectly binding to the target substance and a region capable of binding to at least one of the nucleic acid probes in a solution; (B) binding the target substance to the target substance detection polymer; and (C) detecting the target substance detection polymer to which the target substance is bound, wherein the multiple kinds of nucleic acid probes comprise a nucleic acid probe comprising at least n (n≥3) nucleic acid regions formed of a nucleic acid region X₁, a nucleic acid region X₂, ···, and a nucleic acid region Xₙ from a 5' terminal side in the stated order, and a nucleic acid probe comprising at least n (n≥3) nucleic acid regions formed of a nucleic acid region X₁' complementary to the nucleic acid region X₁, a nucleic acid region X₂' complementary to the nucleic acid region X₂, ···, and a nucleic acid region Xₙ' complementary to the nucleic acid region Xₙ from a 5' terminal side in the stated order.

A second aspect of the method of detecting a target substance of the present invention comprises the steps of: (A) forming a target substance detection polymer by causing multiple kinds of nucleic acid probes for forming a polymer to react with a binding probe comprising a target region capable of directly or indirectly binding to the target substance and a region capable of binding to at least one of the nucleic acid probes in a solution; (B) binding the target substance to the target substance detection polymer; and (C) detecting the target substance detection polymer to which the target substance is bound, wherein the multiple kinds of nucleic acid probes each comprise a base sequence that hybridizes with any other kind of nucleic acid probe as represented by the following formula (I):

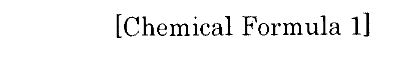

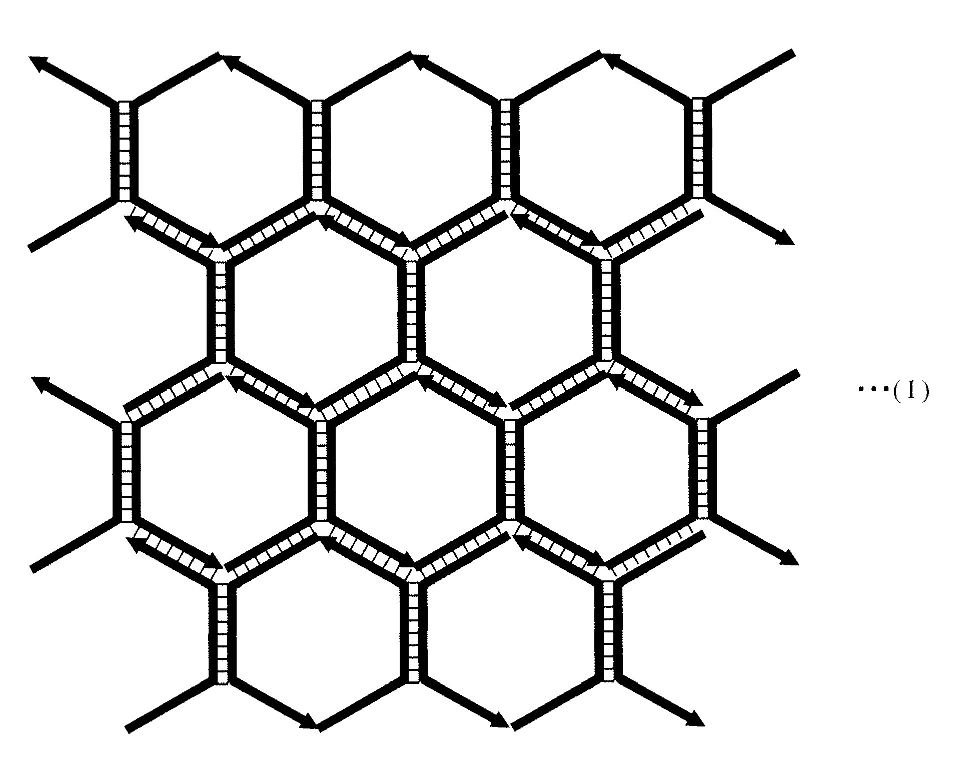

It should be noted that in the formula (I) described above, two straight lines illustrated in a ladder fashion represent nucleic acids that hybridize with each other, and the direction of an arrow represents the direction from the 5' terminal to the 3' terminal. In addition, in the present invention, a region that hybridizes with any other nucleic acid probe is called a complementary region. Although a gap may be present between complementary regions in the nucleic acid probes, the gap is preferably absent.

A third aspect of the method of detecting a target substance of the present invention comprises the steps of: (A) forming a target substance detection polymer by causing multiple kinds of nucleic acid probes for forming a polymer to react with a binding probe comprising a target region capable of directly or indirectly binding to the target substance and a region capable of binding to at least one of the nucleic acid probes in a solution; (B) binding the target substance to the target substance detection polymer; and (C) detecting the target substance detection polymer to which the target substance is bound, wherein the multiple kinds of nucleic acid probes each comprise a base sequence that hybridizes with any other kind of nucleic acid probe as represented by the following formula (II):

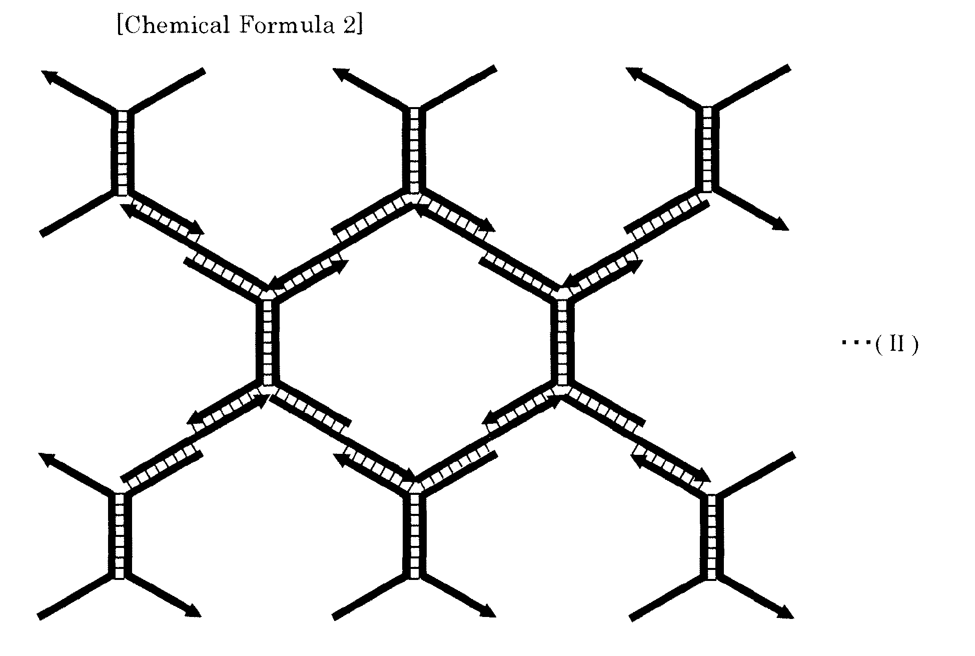

It should be noted that in the formula (II) described above, two straight lines illustrated in a ladder fashion represent nucleic acids that hybridize with each other, and the direction of an arrow represents the direction from the 5' terminal to the 3' terminal. In addition, in the present invention, a region that hybridizes with any other nucleic acid probe is called a complementary region. Although a gap may be present between complementary regions in the nucleic acid probes, the gap is preferably absent.

In the first to third aspects of the method of detecting a target substance of the present invention, the formation step preferably comprises a first hybridization step of causing the multiple kinds of nucleic acid probes to hybridize with each other to form a first polymer and a second hybridization step of causing the first polymer and the binding probe to hybridize with each other to form the target substance detection polymer.
Further, in the first to third aspects of the method of detecting a target substance of the present invention, the formation step may comprise the step of causing the multiple kinds of nucleic acid probes and the binding probe to hybridize with one another simultaneously.

The first to third aspects of the method of detecting a target substance of the present invention further suitably comprise the step of diluting a solution containing the target substance detection polymer after the formation step to prepare a detection solution.

The first aspect of the method of forming a target substance detection polymer of the present invention comprises the step of forming a target substance detection polymer by causing multiple kinds of nucleic acid probes for forming a polymer to hybridize with a binding probe comprising a target region capable of directly or indirectly binding to the target substance and a region capable of binding to at least one of the nucleic acid probes, wherein: the binding probe is free of being bound to the target substance; and the multiple kinds of nucleic acid probes comprise a nucleic acid probe comprising at least n (n≥3) nucleic acid regions formed of a nucleic acid region X₁, a nucleic acid region X₂, ···, and a nucleic acid region Xₙ from a 5' terminal side in the stated order, and a nucleic acid probe having at least n (n≥3) nucleic acid regions formed of a nucleic acid region X₁' complementary to the nucleic acid region X₁, a nucleic acid region X₂' complementary to the nucleic acid region X₂, ···, and a nucleic acid region Xₙ' complementary to the nucleic acid region Xₙ from a 5' terminal side in the stated order.

The second aspect of the method of forming a target substance detection polymer of the present invention comprises the step of forming the target substance detection polymer by causing multiple kinds of nucleic acid probes for forming a polymer to hybridize with a binding probe comprising a target region capable of directly or indirectly binding to the target substance and a region capable of binding to at least one of the nucleic acid probes, wherein: the binding probe is free of being bound to the target substance; and the multiple kinds of nucleic acid probes each comprise a base sequence that hybridizes with any other kind of nucleic acid probe as represented by the following formula (I):

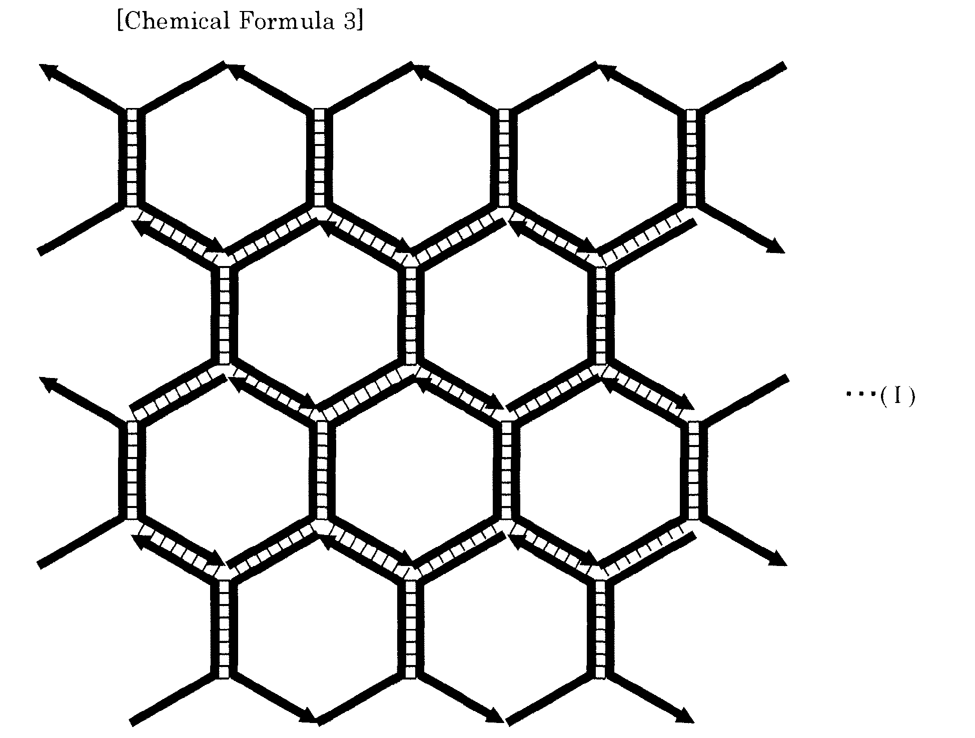

(It should be noted that in the formula (I) described above, two straight lines illustrated in a ladder fashion represent nucleic acids that hybridize with each other).

The third aspect of the method of forming a target substance detection polymer of the present invention comprises the step of forming the target substance detection polymer by causing multiple kinds of nucleic acid probes for forming a polymer to hybridize with a binding probe comprising a target region capable of directly or indirectly binding to the target substance and a region capable of binding to at least one of the nucleic acid probes, in which: the binding probe is free of being bound to the target substance; and the multiple kinds of nucleic acid probes each comprise a base sequence that hybridizes with any other kind of nucleic acid probe as represented by the following formula (II):

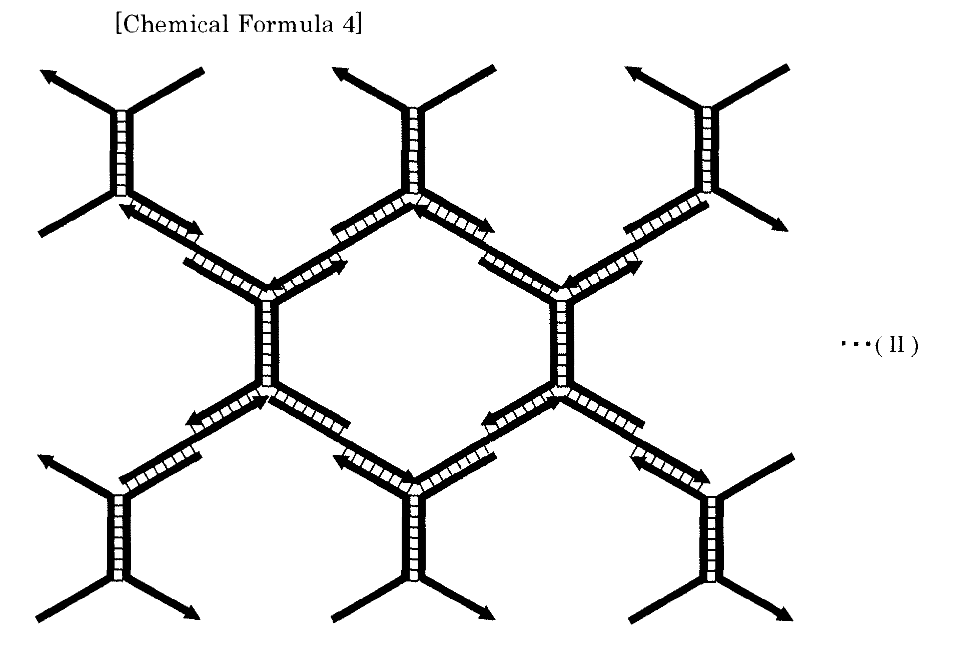

(It should be noted that in the formula (II) described above, two straight lines illustrated in a ladder fashion represent nucleic acids that hybridize with each other.)

In the first to third aspects of the method of forming a target substance detection polymer of the present invention, the step of forming a target substance detection polymer preferably comprises a first hybridization step of causing the multiple kinds of nucleic acid probes to hybridize with each other to form a first polymer and a second hybridization step of causing the first polymer and the binding probe to hybridize with each other to form the target substance detection polymer.
Further, in the first to third aspects of the method of forming a target substance detection polymer of the present invention, the step of forming a target substance detection polymer may comprise the step of causing the multiple kinds of nucleic acid probes and the binding probe to hybridize with one another simultaneously.

In the formation method of the present invention, at least one kind of nucleic acid probe of the multiple kinds of nucleic acid probes is preferably labeled with a labeling substance.

In the formation method of the present invention, the target region of the binding probe preferably comprises a portion capable of specifically binding to the target substance. When the target substance comprises a nucleic acid, the target region, capable of binding to the target substance, of the binding probe preferably comprises a nucleic acid region comprising a base sequence complementary to that of the target nucleic acid.

Further, in the formation method of the present invention, the target substance detection polymer is suitably bound to avidin or biotin through the binding probe.
The order in which the step of binding avidin or biotin to the target substance detection polymer is performed is not particularly limited. For example, the target substance detection polymer to which avidin or biotin is bound is preferably formed with a binding probe obtained by binding avidin or biotin in advance by causing the binding probe to react with the multiple kinds of nucleic acid probes for forming the polymer.
Alternatively, avidin or biotin can be bound during or after the formation of the polymer with a binding probe whose target region is a region to which avidin or biotin can be directly or indirectly bound. When a binding probe to which neither avidin nor biotin is bound is used, the step of forming the target substance detection polymer preferably includes the steps of causing the multiple kinds of nucleic acid probes for forming the polymer to hybridize with the binding probe to form a second polymer, and of binding avidin or biotin to the binding probe of the second polymer.

The first aspect of the target substance detection polymer of the present invention comprises: multiple kinds of nucleic acid probes for forming a polymer; and a binding probe comprising a target region capable of directly or indirectly binding to the target substance and a region capable of binding to at least one of the nucleic acid probes, the target substance detection polymer being formed by causing the multiple kinds of nucleic acid probes to hybridize with the binding probe, in which: the binding probe is free of being bound to the target substance; and the multiple kinds of nucleic acid probes comprise a nucleic acid probe comprising at least n (n≥3) nucleic acid regions formed of a nucleic acid region X₁, a nucleic acid region X₂, ···, and a nucleic acid region Xₙ from a 5' terminal side in the stated order, and a nucleic acid probe having at least n (n≥3) nucleic acid regions formed of a nucleic acid region X₁' complementary to the nucleic acid region X₁, a nucleic acid region X₂' complementary to the nucleic acid region X₂, ···, and a nucleic acid region Xₙ' complementary to the nucleic acid region Xₙ from a 5' terminal side in the stated order.

The second aspect of the target substance detection polymer of the present invention comprises: multiple kinds of nucleic acid probes for forming a polymer; and a binding probe comprising a target region capable of directly or indirectly binding to the target substance and a region capable of binding to at least one of the nucleic acid probes, the target substance detection polymer being formed by causing the multiple kinds of nucleic acid probes to hybridize with the binding probe, in which: the binding probe is free of being bound to the target substance; and the multiple kinds of nucleic acid probes each comprise a base sequence that hybridizes with any other kind of nucleic acid probe as represented by the following formula (I):

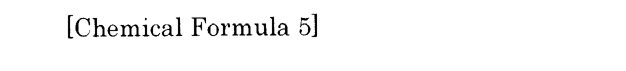

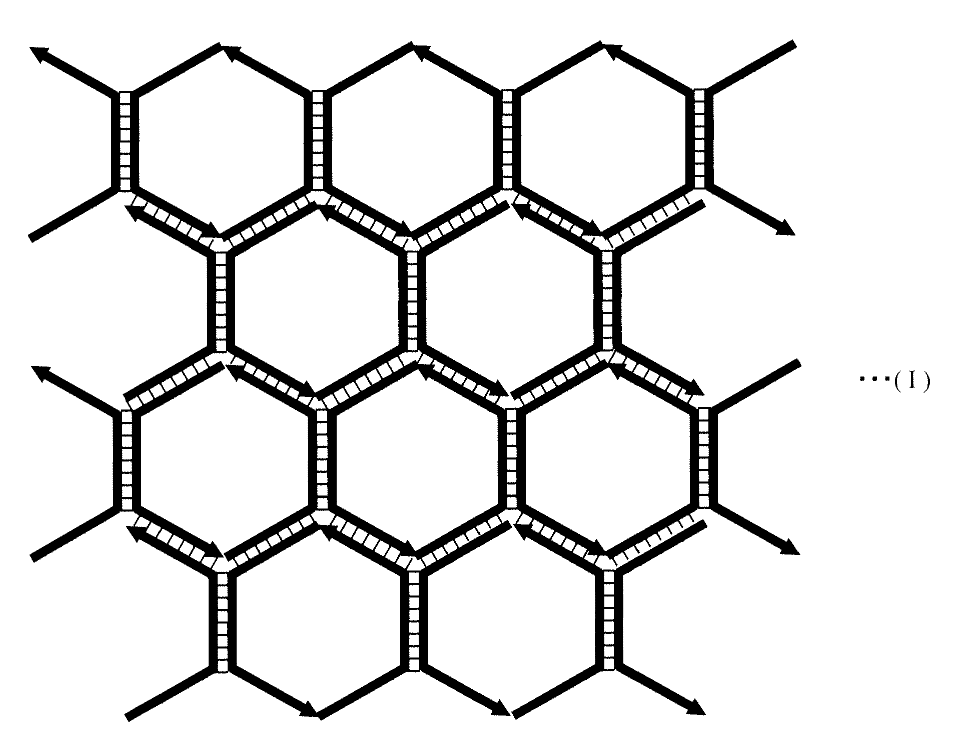

(It should be noted that in the formula (I) described above, two straight lines illustrated in a ladder fashion represent nucleic acids that hybridize with each other).

The third aspect of the target substance detection polymer of the present invention comprises: multiple kinds of nucleic acid probes for forming a polymer; and a binding probe comprising a target region capable of directly or indirectly binding to the target substance and a region capable of binding to at least one of the nucleic acid probes, the target substance detection polymer being formed by causing the multiple kinds of nucleic acid probes to hybridize with the binding probe, in which: the binding probe is free of being bound to the target substance; and the multiple kinds of nucleic acid probes each comprise a base sequence that hybridizes with any other kind of nucleic acid probe as represented by the following formula (II):

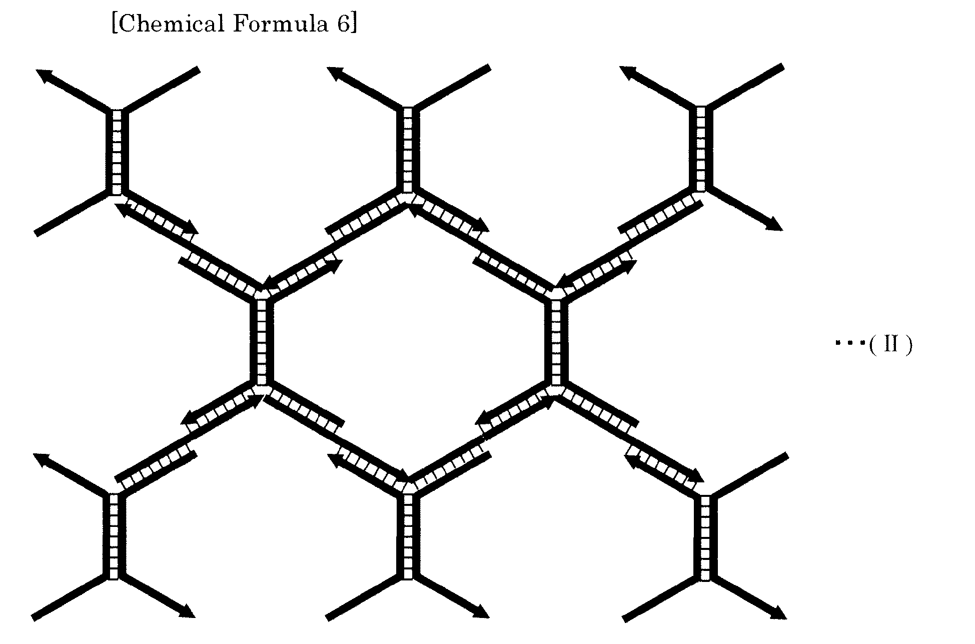

(It should be noted that in the formula (II) described above, two straight lines illustrated in a ladder fashion represent nucleic acids that hybridize with each other.)

In the target substance detection polymer of the present invention, the target region of the binding probe preferably comprises a portion capable of specifically binding to the target substance. When the target substance comprises a nucleic acid, the target region, capable of binding to the target substance, of the binding probe preferably comprises a nucleic acid region comprising a base sequence complementary to that of the target nucleic acid. Further, the target substance detection polymer of the present invention is suitable bound to avidin or biotin through the binding probe.

In the fourth aspect of the target substance detection polymer of the present invention, the polymer is formed by the method of forming a target substance detection polymer of the present invention.

The fourth aspect of the method of detecting a target substance of the present invention comprises the steps of: binding the target substance to the target substance detection polymer of the present invention; and detecting the target substance detection polymer to which the target substance is bound.

In the detection method of the present invention, at least one kind of nucleic acid probe of the multiple kinds of nucleic acid probes is preferably labeled with a labeling substance.

In the detection method of the present invention, the target region of the binding probe preferably comprises a portion capable of specifically binding to the target substance. When the target substance comprises a nucleic acid, the target region of the binding probe suitably comprises a nucleic acid comprising a base sequence complementary to that of the target nucleic acid.
Further, in the detection method of the present invention, the target substance detection polymer and the target substance may be bound to each other through a spacer substance comprising a region capable of binding to the target substance and a region capable of binding to the target region of the binding probe.

In the detection method of the present invention, the target substance detection polymer is preferably bound to avidin or biotin through the binding probe. The target substance detection polymer formed by binding avidin or biotin and the target substance are suitably bound to each other through a bond between biotin and avidin with the target substance detection polymer.

### Effects of the Invention

According to the present invention, restraints on various reaction conditions such as a reaction solution and a reaction temperature can be alleviated, and hence a target substance can be detected with efficiency and a high sensitivity, and in a simple manner. In addition, according to the present invention, the size of a target substance detection polymer can be controlled.

### Brief Description of the Drawings

FIG. 1 is a schematic explanatory view illustrating a first example of multiple kinds of nucleic acid probes used for polymer formation.
FIG. 2 is a schematic explanatory view illustrating a polymer formed with the multiple nucleic acid probes illustrated in FIG. 1.
FIG. 3 is a schematic explanatory view illustrating a second example of the multiple kinds of nucleic acid probes used for polymer formation.
FIG. 4 is a schematic explanatory view illustrating a polymer formed with the multiple nucleic acid probes illustrated in FIG. 3.
FIG. 5 is a schematic explanatory view illustrating another example of a second dimer probe in the multiple nucleic acid probes illustrated in FIG. 3.
FIG. 6 is a schematic explanatory view illustrating a third example of the multiple kinds of nucleic acid probes used for polymer formation.
FIG. 7 is a schematic explanatory view illustrating a fourth example of the multiple kinds of nucleic acid probes used for polymer formation.
FIG. 8 is a schematic explanatory view illustrating a polymer formed with the multiple nucleic acid probes illustrated in FIG. 7.
FIG. 9 is a schematic explanatory view illustrating two groups of dimer probes in a fifth example of the multiple kinds of nucleic acid probes used for polymer formation.
FIG. 10 is a schematic explanatory view illustrating another example of crosslinking probes in the multiple nucleic acid probes illustrated in FIG. 7.
FIG. 11 is a schematic explanatory view illustrating a polymer formed with the dimer probe illustrated in FIG. 7 and the crosslinking probes illustrated in FIG. 10.
FIG. 12 is a schematic explanatory view illustrating two groups of crosslinking probes in the fifth example of the multiple kinds of nucleic acid probes used for polymer formation.
FIG. 13 is a schematic explanatory view illustrating a sixth example of the multiple kinds of nucleic acid probes used for polymer formation.
FIG. 14 is a schematic explanatory view illustrating a polymer formed with the multiple nucleic acid probes illustrated in FIG. 13.
FIG. 15 is a schematic explanatory view illustrating an example of a target substance detection polymer of the present invention.
FIG. 16 is a schematic explanatory view illustrating another example of a target substance detection polymer of the present invention.
FIG. 17 is a graph illustrating the results of Example 6.
FIG. 18 is a graph illustrating the results of Example 8.
FIG. 19 is a graph illustrating the results of Example 9.

### Description of Symbols

10: a first nucleic acid probe, 12: a second nucleic acid probe, 13: a hydrogen bond, 14, 22, and 34: polymers, 20a: a first dimer probe, 20b, 20c, and 20d: second dimer probes, 20e: a third dimer probe, 21a to 21h, 21j, and 21k: dimer formation probes, 30a and 30b: dimer probes, 31a to 31d: dimer formation probes, 32a to 32d: crosslinking probes, 40a and 40b: dimer probes, 41a to 41d: dimer formation probes, 42a to 42d: crosslinking probes, 50a and 50b: binding probes, 52a and 52b: target substance detection polymers, and S: streptavidin.

### Best Mode for carrying out the Invention

Embodiments of the present invention are hereinafter described based on the attached drawings. Because the illustrated examples are just for exemplification, various modifications are of course feasible as long as the modifications do not depart from the technical idea of the present invention.

The present invention relates to the detection of a target substance with a polymer produced by the PALSAR method in advance and having a binding probe bound to itself (in the present invention, a polymer to which the binding probe not bound to the target substance is bound is called a target substance detection polymer).
That is, a method of detecting the target substance of the present invention is characterized by including the steps of preparing a target substance detection polymer, causing the target substance detection polymer and the target substance to react with each other to directly or indirectly bind the target substance detection polymer and the target substance, and detecting the target substance detection polymer to which the target substance is bound.

The target substance detection polymer of the present invention can be formed by causing multiple kinds of nucleic acid probes for forming a polymer to react with a binding probe having a target region capable of directly or indirectly binding to the target substance and a region capable of binding to at least one of the nucleic acid probes in a solution.
The multiple kinds of nucleic acid probes are not particularly limited as long as the nucleic acid probes have base sequences complementary to each other and can form a polymer of the nucleic acid probes by a probe alternation link self-assembly reaction. Specific examples of the nucleic acid probes include three embodiments to be described later (PALSAR I, PALSAR II, and PALSAR III). In the present invention, nucleic acid probes to each of which neither avidin nor biotin is bound are used as the multiple kinds of nucleic acid probes for forming a polymer.

### (PALSAR I)

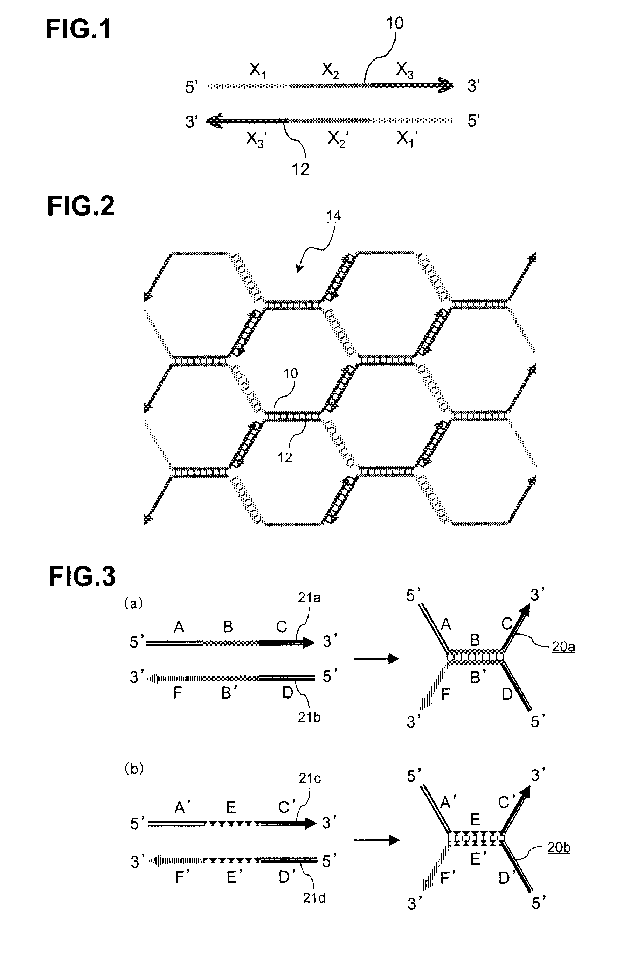

FIG. 1 is a schematic explanatory view illustrating a first example of the multiple kinds of nucleic acid probes used for polymer formation. FIG. 2 is a schematic explanatory view illustrating a polymer formed with the multiple nucleic acid probes illustrated in FIG. 1.
Examples of the multiple kinds of nucleic acid probes used for polymer formation include two kinds of nucleic acid probes, i.e., a first nucleic acid probe 10 having at least n (n≥3) nucleic acid regions formed of a nucleic acid region X₁, a nucleic acid region X₂, ···, and a nucleic acid region Xₙ from the 5' terminal side in the stated order, and a second nucleic acid probe 12 having at least n (n≥3) nucleic acid regions formed of a nucleic acid region X₁' complementary to the nucleic acid region X₁, a nucleic acid region X₂' complementary to the nucleic acid region X₂, ···, and a nucleic acid region Xₙ' complementary to the nucleic acid region Xₙ from the 5' terminal side in the stated order as illustrated in FIG. 1. It should be noted that FIG. 1 illustrates an example of the case where n equals 3. Although n is not particularly limited as long as n is equal to or larger than 3, n is preferably 3 or more and 5 or less, or more preferably 3.

By causing the two kinds of nucleic acid probes 10 and 12 to hybridize with each other, the two kinds of nucleic acid probes 10 and 12 are self assembled as illustrated in FIG. 2 to form a polymer 14 of the nucleic acid probes. It should be noted that, when multiple kinds of nucleic acid probes having a number n of complementary nucleic acid regions of 3 are used in PALSAR I, the multiple kinds of nucleic acid probes each hybridize with any other kind of nucleic acid probe as represented by the formula (I) (see FIG. 2 and the formula (I)).

### (PALSAR II)

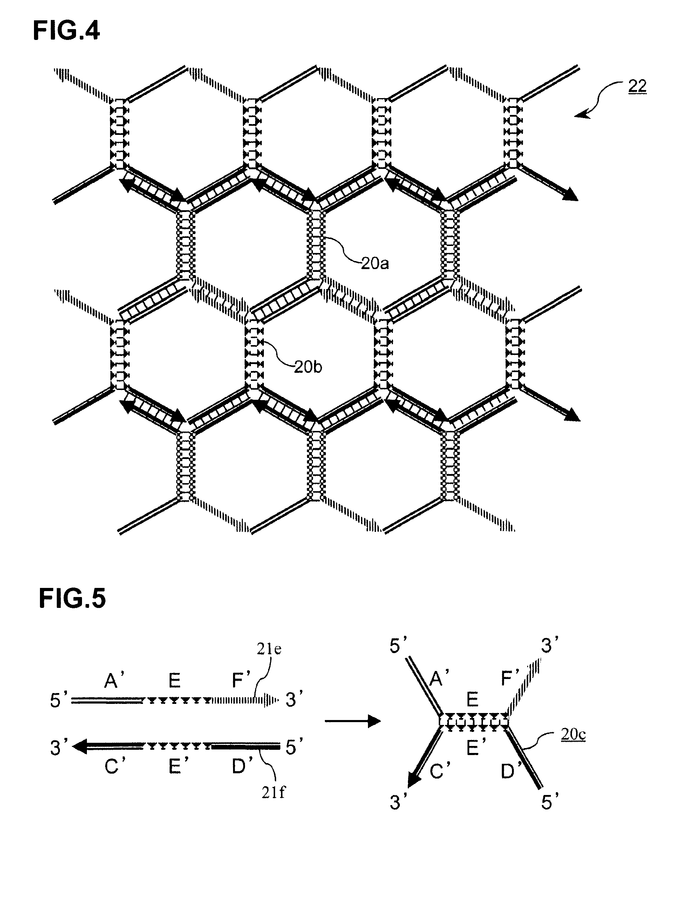

FIG. 3 is a schematic explanatory view illustrating a second example of the multiple kinds of nucleic acid probes used for polymer formation. FIG. 4 is a schematic explanatory view illustrating a polymer formed with the multiple nucleic acid probes illustrated in FIG. 3.
As illustrated in FIG. 3, examples of the multiple kinds of nucleic acid probes used for polymer formation include multiple dimer probes for forming a polymer, and dimer formation probes for forming the dimer probes. The multiple dimer probes are constituted so that each 5'-side region of one dimer probe of the multiple dimer probes is complementary to one of the 5' side regions of any other dimer probe and each 3'-side region of one dimer probe of the multiple dimer probes is complementary to one of the 3'-side regions of any other dimer probe, and the dimer probes can self-assemble by themselves to form an assembly (probe polymer). For example, the nucleic acid probes described in Patent Document 3 are used. The multiple kinds of nucleic acid probes of PALSAR II each have a base sequence that hybridizes with any other kind of nucleic acid probe as represented by the formula (I) (see FIG. 4 and the formula (I)).

FIG. 3 illustrates an example of the case where two groups of dimer probes (a first dimer probe 20a and a second dimer probe 20b) are used.
As illustrated in FIG. 3(a), the first dimer probe 20a is formed by causing two kinds of single-stranded nucleic acid probes (a first dimer formation probe 21a and a second dimer formation probe 21b) to hybridize with each other. The first dimer formation probe 21a includes three regions, i.e., the 5'-side region (region A), the central region (region B), and the 3'-side region (region C), and the second dimer formation probe 21b includes three regions, i.e., the 5'-side region (region D), the central region (region B'), and the 3'-side region (region F). The first dimer formation probe 21a and the second dimer formation probe 21b are such that the central regions (regions B and B') are complementary to each other, and the 3'-side regions (regions C and F) and the 5'-side regions (regions A and D) are noncomplementary to each other.

As illustrated in FIG. 3(b), the second dimer probe 20b is formed by causing two kinds of single-stranded nucleic acid probes (a third dimer formation probe 21c and a fourth dimer formation probe 21d) to hybridize with each other. The third dimer formation probe 21c includes three regions, i.e., the 5'-side region (region A'), the central region (region E), and the 3'-side region (region C'), and the fourth dimer formation probe 21d includes three regions, i.e., the 5'-side region (region D'), the central region (region E'), and the 3'-side region (region F'). The third dimer formation probe 21c and the fourth dimer formation probe 21d are such that the central regions (regions E and E') are complementary to each other, and the 3'-side regions (regions C' and F') and the 5'-side regions (regions A' and D') are noncomplementary to each other.
It should be noted that, in the present invention, the region A' means a region having a base sequence complementary to that of the region A, the region C' means a region having a base sequence complementary to that of the region C, the region D' means a region having a base sequence complementary to that of the region D, and the region F' means a region having a base sequence complementary to that of the region F.

The 5'-side regions (regions A and D) of the first dimer probe 20a are complementary to the 5'-side regions (regions A' and D') of the second dimer probe 20b, and the 3'-side regions (regions C and F) of the first dimer probe 20a are complementary to the 3'-side regions (regions C' and F') of the second dimer probe 20b. By causing the first and second dimer probes 20a and 20b to hybridize with each other, a polymer 22 of the nucleic acid probes is formed (FIG. 4).
In the present invention, the dimer probes are preferably used, though the dimer formation probes before the formation of the dimer probes may be used instead of the dimer probes.

FIG. 3 illustrates an example in which the 5'-side region and 3'-side region of the first dimer formation probe 21a are complementary to the 5'-side region and 3'-side region of the third dimer formation probe 21c, respectively, and the 5'-side region and 3'-side region of the second dimer formation probe 21b are complementary to the 5'-side region and 3'-side region of the fourth dimer formation probe 21d, respectively. In the present invention, however, the dimer probes have only to be such that the 5'-side region of one dimer probe is complementary to the 5'-side region of any other dimer probe and the 3'-side region of one dimer probe is complementary to the 3'-side region of any other dimer probe.

FIG. 5 is a schematic explanatory view illustrating another example of the second dimer probe used together with the first dimer probe 20a illustrated in FIG. 3.
As illustrated in FIG. 5, a dimer probe 20c formed by causing a dimer formation probe 21e whose 5'-side region is a region (region A') complementary to the 5'-side region of the first dimer formation probe 21a and whose 3'-side region is a region (region F') complementary to the 3'-side region of the second dimer formation probe 21b and a dimer formation probe 11f whose 5'-side region is a region (region D') complementary to the 5'-side region of the second dimer formation probe 21b and whose 3'-side region is a region (region C') complementary to the 3'-side region of the first dimer formation probe 21a to hybridize with each other can be used as the second dimer probe.

Although FIG. 1 illustrates an example in which two kinds of dimer probes are used, a larger number of kinds of dimer probes can also be used when contrivance is made on a positional relationship between complementary regions (Patent Document 3).

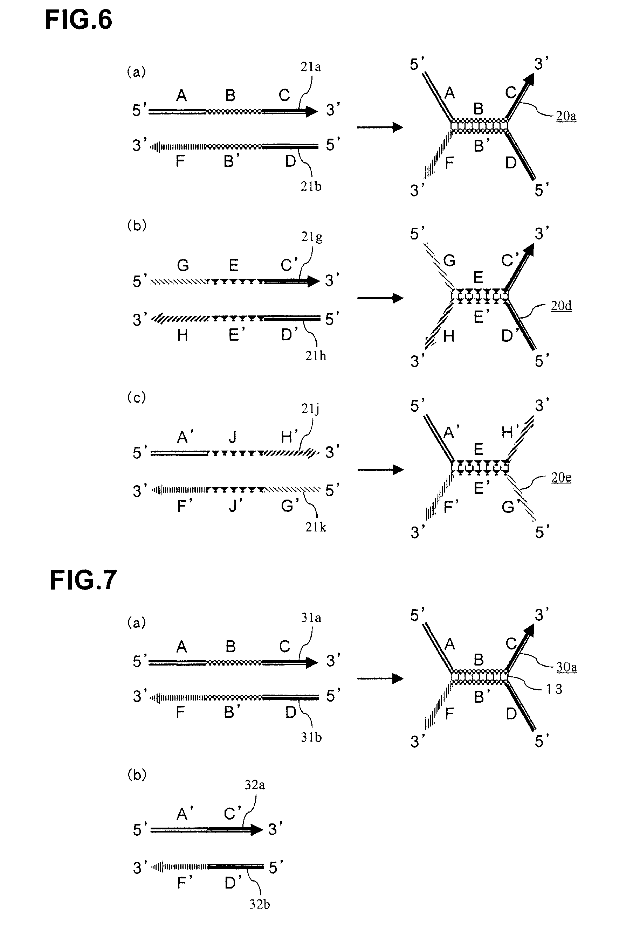

FIG. 6 is a schematic explanatory view illustrating a third example of the multiple kinds of nucleic acid probes used for polymer formation, and is a schematic explanatory view illustrating an example of the case where three groups of dimer probes (the first dimer probe 20a, a second dimer probe 20d, and a third dimer probe 20e) are used.
In FIG. 6(a), the first dimer probe 20a is constituted as in FIG. 3(a).
In FIG. 6(b), the second dimer probe 20d is formed by causing two kinds of single-stranded nucleic acid probes (dimer formation probes 21g and 21h) to hybridize with each other. The dimer formation probe 21g includes three regions, i.e., the 5'-side region (region G), the central region (region E), and the 3'-side region (region C'), and the 3'-side region (region C') is complementary to the 3'-side region (region C) of the first dimer probe 20a. The dimer formation probe 21h includes three regions, i.e., the 5'-side region (region D'), the central region (region E'), and the 3'-side region (region H), and the 5'-side region (region D') is complementary to the 5'-side region (region D) of the first dimer probe 20a.

In FIG. 6(c), the third dimer probe 20e is formed by causing two kinds of single-stranded nucleic acid probes (dimer formation probes 21j and 21k) to hybridize with each other. The dimer formation probe 21j includes three regions, i.e., the 5'-side region (region A'), the central region (region J), and the 3'-side region (region H'), the 5'-side region (region A') is complementary to the 5'-side region (region A) of the first dimer probe 20a, and the 3'-side region (region H') is complementary to the 3'-side region (region H) of the second dimer probe 20d. The dimer formation probe 21k includes three regions, i.e., the 5'-side region (region G'), the central region (region J'), and the 3'-side region (region F'), the 5'-side region (region G') is complementary to the 5'-side region (region G) of the second dimer probe 20d, and the 3'-side region (region F') is complementary to the 3'-side region (region F) of the first dimer probe 20a. It should be noted that, in the figure, the region J' is a region complementary to the region J.

That is, in FIG. 6, the dimer probes are constituted so that one 5'-side region, and one 3'-side region, of the first dimer probe are complementary to one 5'-side region, and one 3'-side region, of the second dimer probe, the other 5'-side region, and the other 3'-side region, of the first dimer probe are complementary to one 5'-side region, and one 3'-side region, of the third dimer probe, and the other 5'-side region, and the other 3'-side region, of the second dimer probe are complementary to the other 5'-side region, and the other 3'-side region, of the third dimer probe.

The multiple kinds of dimer probes constituted so that each 3'-side region of each dimer probe is complementary to one of the 3'-side regions of any other dimer probe and each 5'-side region of each dimer probe is complementary to one of the 5'-side regions of any other dimer probe as illustrated in FIG. 6 are used, and these multiple kinds of dimer probes are caused to hybridize with each other. Thus, a polymer as an assembly of the dimer probes is formed.
Although a combination of dimer probes having a complementary relationship is not particularly limited in the present invention, the constitution is preferably performed so that one 3'-side region and one 5'-side region in each dimer probe are complementary to one 3'-side region and one 5'-side region in another dimer probe, respectively as illustrated in FIG. 6.

### (PALSAR III)

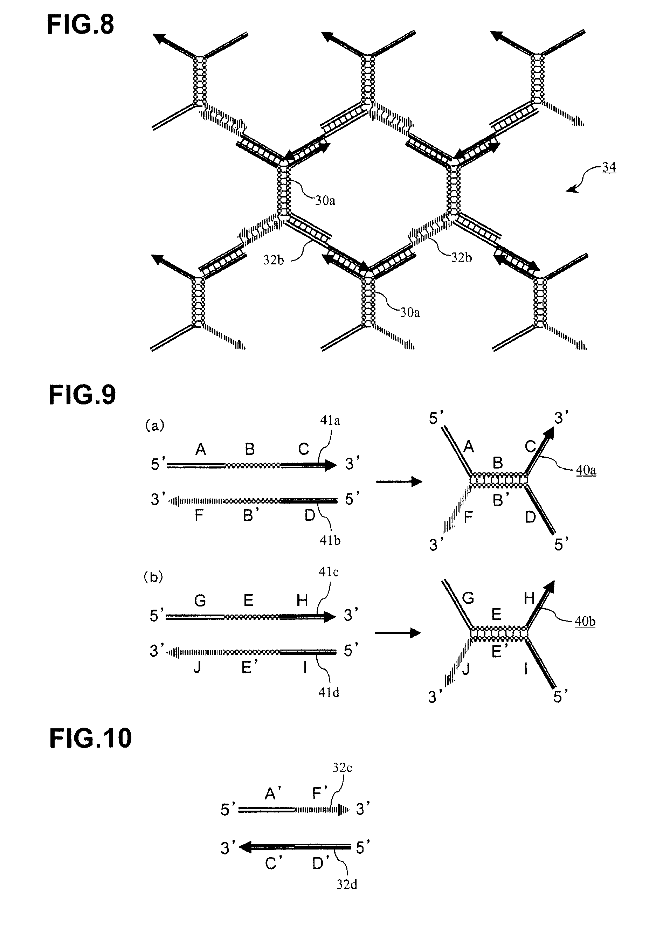

FIG. 7 is a schematic explanatory view illustrating a fourth example of the multiple kinds of nucleic acid probes used for polymer formation. FIG. 8 is a schematic explanatory view illustrating a polymer formed with the multiple nucleic acid probes illustrated in FIG. 7.
Examples of the multiple kinds of nucleic acid probes used for polymer formation include one or more groups of a pair of dimer formation probes or of a dimer probe formed of the pair of dimer formation probes, and one or more kinds of crosslinking probes as illustrated in FIG. 7. The multiple kinds of nucleic acid probes of PALSAR III each have a base sequence that hybridizes with any other kind of nucleic acid probe as represented by the formula (II) (see FIG. 8 and the formula (II)).

FIG. 7 illustrates a first example of the case where one group of a pair of dimer formation probes and one group of a pair of crosslinking probes are used.
FIG. 7(a) illustrates one group of the pair of dimer formation probes (a first dimer formation probe 31a and a second dimer formation probe 31b), and a dimer probe 30a formed of the pair of dimer formation probes 31a and 31b.
As illustrated in FIG. 7(a), the pair of dimer formation probes is formed of two kinds of single-stranded nucleic acid probes (the first dimer formation probe 31a and the second dimer formation probe 31b), and each of the dimer formation probes 31a and 31b includes at least three regions, i.e., a central region, a 5'-side region positioned on a 5' side relative to the central region, and a 3'-side region positioned on a 3' side relative to the central region. In FIG 7, the 5'-side region, central region, and 3'-side region of the first dimer formation probe 31a are represented as a region A, a region B, and a region C, respectively, and the 5'-side region, central region, and 3'-side region of the second dimer formation probe 31b are represented as a region D, a region B', and a region F, respectively. The central regions (regions B and B') of the first dimer formation probe 31a and the second dimer formation probe 31b are complementary to each other, and all the four regions, i.e., 5'-side regions (regions A and D) and 3'-side regions (regions C and F) of the first dimer formation probe 31a and the second dimer formation probe 31b are noncomplementary to each other. By causing both the probes to hybridize with each other, one group of a dimer probe 30a is formed. In the figure, reference symbol 13 represents a hydrogen bond.

In the present invention, a dimer probe formed by causing the pair of dimer formation probes to hybridize with each other in advance may be used, or the dimer formation probes may be used as they are. The dimer probe is preferably used.

When multiple groups of the pair of dimer formation probes are used, each group of the pair of dimer formation probes is constituted as in the case of the one group described above. The multiple groups of the pair of dimer formation probes result in the formation of the same number of groups of the dimer probe.

FIG. 9 is a schematic explanatory view illustrating an example of two groups of a pair of dimer formation probes in a fifth example of the multiple kinds of nucleic acid probes used for polymer formation and two groups of dimer probes each formed of the pair of dimer formation probes. FIG. 9(a) illustrates a first group of a pair of dimer formation probes (a first dimer formation probe 41a and a second dimer formation probe 41b) and a dimer probe 40a formed of the pair of dimer formation probes 41a and 41b, and FIG. 9(b) illustrates a second group of a pair of dimer formation probes (a first dimer formation probe 41c and a second dimer formation probe 41d) and a dimer probe 40b formed of the pair of dimer formation probes 41c and 41d.
In FIG. 9, the 5'-side region, central region, and 3'-side region of the first dimer formation probe 41a of the first group are represented as a region A, a region B, and a region C, respectively, and the 5'-side region, central region, and 3'-side region of the second dimer formation probe 41b of the first group are represented as a region D, a region B', and a region F, respectively. Similarly, the 5'-side region, central region, and 3'-side region of the first dimer formation probe 41c of the second group are represented as a region G, a region E, and a region H, respectively, and the 5'-side region, central region, and 3'-side region of the second dimer formation probe 41d of the second group are represented as a region I, a region E', and a region J, respectively. The central regions (regions B and B' or regions E and E') of the first and second dimer formation probes of each group are complementary to each other, and all the four regions, i.e., 5'-side regions and 3'-side regions (regions A, C, D, and F or regions G, H, I, and J) of each pair of the first and second dimer formation probes are noncomplementary to each other. By causing the respective groups of the pair of dimer formation probes 41a, 41b, 41c, and 41d to hybridize with each other, the two groups of dimer probes 40a and 40b are formed. In the present invention, all the 3'-side regions and 5'-side regions of the dimer formation probes used in PALSAR III are preferably noncomplementary to each other.

In the present invention, crosslinking probes are one or more kinds of single-stranded nucleic acid probes capable of crosslinking dimer probes formed of dimer formation probes, and each include at least two regions. Of the two regions, a region positioned on a 5' side is called a 5'-side region and a region positioned on a 3' side is called a 3'-side region. In the present invention, the 5'-side region of each dimer formation probe is complementary to one of the 5'-side regions of the crosslinking probes, and the 3'-side region of each dimer formation probe is complementary to one of the 3'-side regions of the crosslinking probes. With such constitution, the crosslinking probes bind to dimer formation probes so as to crosslink multiple dimer probes of one or more kinds formed of the dimer formation probes, and hence an assembly of the probes (probe polymer) can be formed.

When one group of a pair of dimer formation probes is used, one group of a pair of crosslinking probes is preferably used. One group of a pair of dimer formation probes (a first dimer formation probe and a second dimer formation probe) and one group of a pair of crosslinking probes (a first crosslinking probe and a second crosslinking probe) are constituted so that each 5'-side region of the pair of dimer formation probes is complementary to one of the 5'-side regions of the pair of crosslinking probes, each 5'-side region of the pair of crosslinking probes is complementary to one of the 5'-side regions of the pair of dimer formation probes, each 3'-side region of the pair of dimer formation probes is complementary to one of the 3'-side regions of the pair of crosslinking probes, and each 3'-side region of the pair of crosslinking probes is complementary to one of the 3'-side regions of the pair of dimer formation probes.

FIG. 7(b) is a schematic explanatory view illustrating an example of one group of a pair of crosslinking probes (a first crosslinking probe 32a and a second crosslinking probe 32b) used together with the pair of dimer formation probes 31a and 31b illustrated in FIG. 7(a).
The crosslinking probes used together with one group of the pair of dimer formation probes 31a and 31b illustrated in FIG. 7(a) are suitably, for example, the following pair of crosslinking probes. That is, as illustrated in FIG. 7(b), the 5'-side region of the first crosslinking probe 32a is complementary to the 5'-side region (region A) of the first dimer formation probe 31a, the 3'-side region of the first crosslinking probe 32a is complementary to the 3'-side region (region C) of the first dimer formation probe 31a, the 5'-side region of the second crosslinking probe 32b is complementary to the 5'-side region (region D) of the second dimer formation probe 31b, and the 3'-side region of the second crosslinking probe 32b is complementary to the 3'-side region (region F) of the second dimer formation probe 31b. By causing the dimer formation probes 13a-31d illustrated in FIG. 7(a) and the crosslinking probes 32a and 32b illustrated in FIG. 7(b) to hybridize with each other, a polymer 34 is formed(FIG. 8).

FIG. 7 illustrates an example in which the 5'-side region and 3'-side region of the first dimer formation probe 31a are complementary to the 5'-side region and 3'-side region of the first crosslinking probe 32a, respectively, and the 5'-side region and 3'-side region of the second dimer formation probe 31b are complementary to the 5'-side region and 3'-side region of the second crosslinking probe 32b, respectively. In the present invention, the 5'-side region of one dimer formation probe has only to be complementary to the 5'-side region of one crosslinking probe and the 3'-side region of one dimer formation probe has only to be complementary to the 3'-side region of one crosslinking probe.

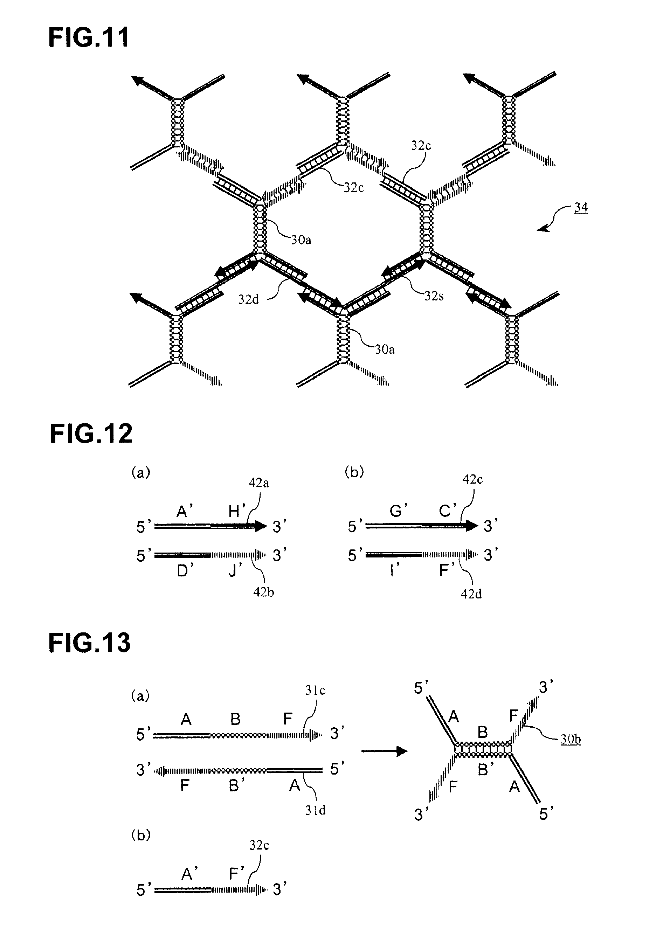

FIG. 10 is a schematic explanatory view illustrating another example of one group of a pair of crosslinking probes (a first crosslinking probe 32c and a second crosslinking probe 32d) used together with the pair of dimer formation probes 31a and 31b illustrated in FIG. 7.
As illustrated in FIG. 10, one of the other examples of the crosslinking probes is such a pair of crosslinking probes that the 5'-side region of the first crosslinking probe 32c is complementary to the 5'-side region (region A) of the first dimer formation probe 31a, the 3'-side region of the first crosslinking probe 32c is complementary to the 3'-side region (region F) of the second dimer formation probe 31b, the 5'-side region of the second crosslinking probe 32d is complementary to the 5'-side region (region D) of the second dimer formation probe 31b, and the 3'-side region of the second crosslinking probe 32d is complementary to the 3'-side region (region C) of the first dimer formation probe 31b. By causing the dimer formation probes 31a-31d illustrated in FIG. 7(a) and the crosslinking probes 32c and 32d illustrated in FIG. 10 to hybridize with each other, the polymer 34 is formed(FIG. 11).

When multiple groups of a pair of dimer formation probes are used, there are preferably used the same number of groups of a pair of crosslinking probes as the number of the multiple groups of the pair of the dimer formation probes. To be specific, n groups (where n represents an integer of 2 or more) of the pair of dimer formation probes (that is, 2n dimer formation probes) and n groups of the pair of crosslinking probes (that is, 2n crosslinking probes) are suitably used, and constitution is suitably performed so that the 5'-side region of each dimer formation probe is complementary to one of the 5'-side regions of the crosslinking probes, the 5'-side region of each crosslinking probe is complementary to one of the 5'-side regions of the dimer formation probes, the 3'-side region of each dimer formation probe is complementary to one of the 3'-side regions of the crosslinking probes, and the 3'-side region of each crosslinking probe is complementary to one of the 3'-side regions of the dimer formation probes.

FIG. 12 is a schematic explanatory view illustrating an example of two groups of a pair of crosslinking probes 42a-42d used together with the two groups of the pair of dimer formation probes 41a-41d illustrated in FIG. 9. FIG. 12(a) illustrates a first group of the pair of crosslinking probes (the first crosslinking probe 42a and the second crosslinking probe 42b), and FIG. 12(b) illustrates a second group of the pair of crosslinking probes (the first crosslinking probe 42c and the second crosslinking probe 42d).
As illustrated in FIG. 12, two groups of a pair of crosslinking probes (that is, four kinds of crosslinking probes) are suitably used as the crosslinking probes used together with the two groups of the pair of dimer formation probes 41a-41d illustrated in FIG. 9, and constitution is suitably performed so that the 5'-side region of each dimer formation probe is complementary to one of the 5'-side regions of the crosslinking probes, the 5'-side region of each crosslinking probe is complementary to one of the 5'-side regions of the dimer formation probes, the 3'-side region of each dimer formation probe is complementary to one of the 3'-side regions of the crosslinking probes, and the 3'-side region of each crosslinking probe is complementary to one of the 3'-side regions of the dimer formation probes.

To be specific, such two groups of a pair of crosslinking probes as illustrated in FIG. 12 are suitable. That is, the 5'-side region of the first crosslinking probe 42a of the first group is complementary to the 5'-side region (region A) of the first dimer formation probe 41a of the first group, the 5'-side region of the second crosslinking probe 42b of the first group is complementary to the 5'-side region (region D) of the second dimer formation probe 41b of the first group, the 5'-side region of the first crosslinking probe 42c of the second group is complementary to the 5'-side region (region G) of the first dimer formation probe 41c of the second group, the 5'-side region of the second crosslinking probe 42d of the second group is complementary to the 5'-side region (region D) of the second dimer formation probe 41d of the second group, the 3'-side region of the first crosslinking probe 42a of the first group is complementary to one of the 3'-side regions of the four kinds of dimer formation probes 41a-41d (FIG. 12 illustrates the case where the region is complementary to the region H), the 3'-side region of the second crosslinking probe 42b of the first group is complementary to one of the 3'-side regions of the four kinds of dimer formation probes 41a-41d excluding that selected by the first crosslinking probe 42a of the first group (FIG 12 illustrates the case where the region is complementary to the region J), the 3'-side region of the first crosslinking probe 22c of the second group is complementary to one of the 3'-side regions of the four kinds of dimer formation probes 41a-41d excluding those selected by the first and second crosslinking probes 42a-42b of the first group (FIG. 12 illustrates the case where the region is complementary to the region C), and the 3'-side region of the second crosslinking probe 42d of the second group is complementary to the remaining one of the 3'-side regions of the four kinds of dimer formation probes 41a-41d excluding those selected by the first and second crosslinking probes 42a-42b of the first group, and the first crosslinking probe 42c of the second group (FIG. 12 illustrates the case where the region is complementary to the region F). By causing the dimer formation probes 41a-41d illustrated in FIG. 9 and the crosslinking probes 42a-42d illustrated in FIG. 12 to hybridize with each other, the polymer 34 is formed.

Although FIG. 12 illustrates the case where the 3'-side region of the first crosslinking probe 42a of the first group is complementary to the 3'-side region of the first dimer formation probe 41c of the second group, the 3'-side region of the second crosslinking probe 42b of the first group is complementary to the 3'-side region of the second dimer formation probe 41b of the second group, the 3'-side region of the first crosslinking probe 42c of the second group is complementary to the 3'-side region of the first dimer formation probe 41a of the first group, and the 3'-side region of the second crosslinking probe 42d of the second group is complementary to the 3'-side region of the second dimer formation probe 41b of the first group, a combination of the 3'-side regions of dimer formation probes complementary to the 3'-side regions of the respective crosslinking probes is not limited.

In the present invention, noncomplementary base sequences are not limited as long as the base sequences do not hybridize with each other, and identical base sequences are included in the noncomplementary base sequences.

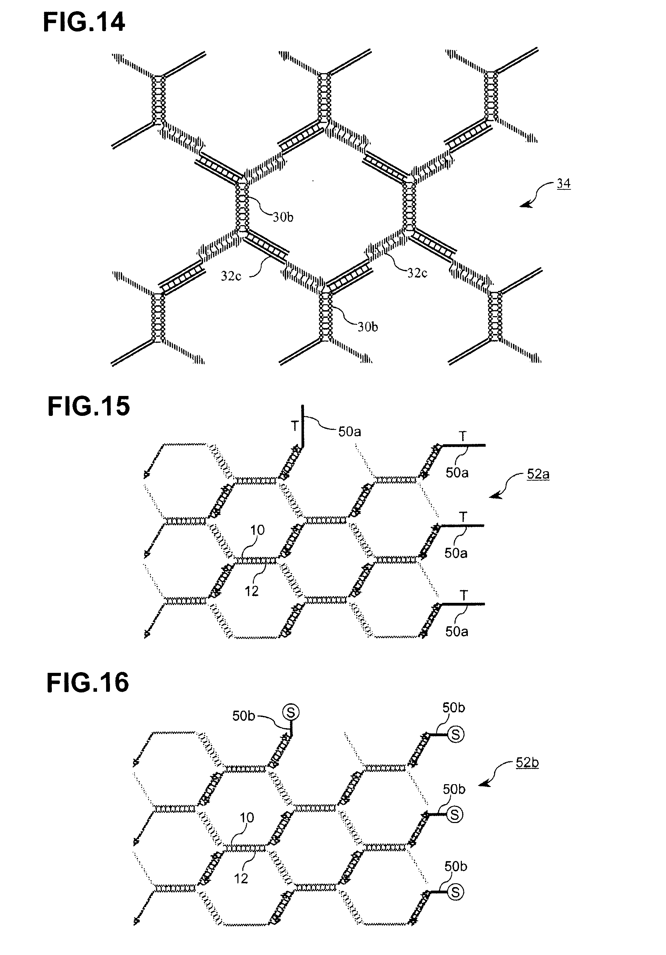

FIG. 13 is a schematic explanatory view illustrating a sixth example of the multiple kinds of nucleic acid probes used for polymer formation, and is a schematic explanatory view illustrating a second example of one group of a pair of dimer formation probes and one kind of a pair of crosslinking probes. In FIG. 13(a), reference symbol 30b represents a dimer probe, and an example in which a dimer is formed with two kinds of dimer formation probes 31c and 31d identical to each other in base sequence of each of a 3'-side region and a 5'-side region is illustrated. That is, in the dimer probe 30a of FIG. 7, the regions A and D are identical to each other in base sequence, and the regions C and F are also identical to each other in base sequence. With such constitution, as illustrated in FIG. 13(b), a pair of crosslinking probes used together with the dimer probe 30b is formed of identical probes, and hence one kind of crosslinking probe 32c is used. By causing the dimer formation probes 31c-31d illustrated in FIG. 13 and the crosslinking probe 32c to hybridize with each other, the polymer 34 is formed (FIG. 14).

The lengths of the respective complementary regions of the respective probes in the multiple kinds of nucleic acid probes used for polymer formation of the present invention are each at least 5 bases, preferably at least 8 bases, more preferably 10 bases to 100 bases, or still more preferably 12 to 30 bases in terms of the number of bases. In addition, complementary regions in the respective probes are desirably identical to each other in length.

The base sequences of the respective regions of the multiple kinds of nucleic acid probes used for polymer formation of the present invention are not particularly limited as long as predetermined regions are constituted to have complementary base sequences so that a polymer is formed. Bases at both terminals of each region are each preferably guanine or cytosine. When the bases at both terminals of each region are each guanine or cytosine, a reaction time can be shortened, and further, a stable probe polymer can be formed at a lower reaction temperature. As a result, workability and detection sensitivity can be improved.

The above nucleic acid probes are generally constituted of DNA or RNA, and may be constituted of nucleic acid analogs. Examples of the nucleic acid analogs include Peptide Nucleic Acid (PNA, see WO 92/20702 and the like) and Locked Nucleic Acid (LNA, see Koshkin AA et al. Tetrahedron 1998. 54, 3607-3630., Koshkin AA et al. J. Am. Chem. Soc. 1998. 120, 13252-13253., Wahlestedt C et al. PNAS. 2000. 97, 5633-5638., etc.). In addition, although multiple nucleic acid probes are generally constituted of the same kind of nucleic acids, a DNA probe and an RNA probe may, for example, constitute a pair. That is, kinds of nucleic acids of the probes can be selected from DNA, RNA, and a nucleic acid analog (such as PNA and LNA). Besides, constitution of nucleic acids in one probe is not limited to constitution of one kind of nucleic acid such as only DNA, and, for example, an oligonucleotide probe (chimeric probe) constituted of DNA and RNA can be used as required. Such a chimeric probe is included in the present invention.

Those probes can be synthesized by a known method. In case of DNA probes, for example, probes can be synthesized by a Phosphoamidide method using a DNA synthesizer 394 type manufactured by Applied Biosystem Inc. In addition, a phosphate triester method, an H-phosphonate method, a thiophosphonate method, and the like can be exemplified as other methods, and the probes synthesized by any method can be used.

The binding probe used in the present invention has a target region T capable of directly or indirectly binding to the target substance and a region capable of binding to at least one of the nucleic acid probes, and the binding probe suitably has the target region T and a base sequence identical to part or the entirety of that of one nucleic acid probe of the multiple nucleic acid probes used for polymer formation. The target region is preferably positioned at an end of the binding probe.

In the present invention, both the binding probe and the target substance may be directly bound to each other, or may be indirectly bound to each other through any other substance. The target region T comprehends not only a region capable of directly binding to the target substance but also a region capable of indirectly binding to the target substance through any other substance.
The target region T can be appropriately selected depending on the target substance. When the target region T is constituted to have a portion capable of specifically binding to the target substance or to have a site capable of directly or indirectly binding to a substance capable of specifically binding to the target substance, the target substance detection polymer and the target substance can be specifically bound to each other. To be specific, when the target substance is a nucleic acid, the target region T is preferably constituted to have a base sequence complementary to that of the target nucleic acid. When the target substance is a protein such as an antigen, it is preferred that a substance that specifically binds to the target substance, such as an antibody, be directly or indirectly bound.

In addition, a spacer substance having a site capable of binding to the target substance and a site capable of binding to the target region T is suitably used for indirectly binding the target substance and the binding probe through the spacer substance. Although means for binding the spacer substance and the binding probe is not limited, for example, a method involving the utilization of such a complementary bond between nucleic acids that one region has a poly T sequence (or a poly G sequence) and the other region has a poly A sequence (or a poly C sequence), a method involving the utilization of a bond between biotin and avidin, a method involving the utilization of a bond between an antigen and an antibody, or a method involving the utilization of a bond between a ligand and a receptor is suitable.

In the present invention, the target region T is suitably a region to which avidin or biotin is bound or a region S to which avidin or biotin can be directly or indirectly bound so that a target substance detection polymer to which avidin is bound or a target substance detection polymer to which biotin is bound may be formed, and the target substance detection polymer and the target substance are suitably detected through a bond between biotin and avidin. The target substance detection polymer to which avidin is bound is more suitably used as the target substance detection polymer. When the target substance detection polymer to which avidin is bound is used, the target substance detection polymer and the target substance are bound to each other through biotin. When the target substance detection polymer to which biotin is bound is used, the target substance detection polymer and the target substance are bound to each other through avidin.

When the target substance detection polymer to which avidin is bound is used as the target substance detection polymer, a method of binding avidin to the binding probe is not particularly limited. For example, it is preferred that a probe having a region capable of binding to at least one of the nucleic acid probes for forming a polymer be labeled with biotin, and avidin be bound through biotin. Alternatively, a probe having a region capable of binding to at least one of the nucleic acid probes may be modified with, for example, NH₂, COOH, or SH, and avidin may be chemically bound to the resultant.

Avidin used in the present invention is a protein having such activity that biotin is bound, and examples of avidin include streptavidin and avidin (derived from albumen). Of those, streptavidin is more preferred.

Although the time point at which avidin is bound to the binding probe is not limited, a method involving using the binding probe obtained by binding avidin in advance and causing the binding probe and a nucleic acid probe to react with each other to form a target substance detection polymer to which avidin is bound, or a method involving using the binding probe to which avidin is not bound (such as the probe to which biotin is bound) to form a polymer formed of the nucleic acid probe and the binding probe (such as a polymer to which biotin is bound) and causing avidin to react after the formation to bind avidin to the polymer is suitable.
When the binding probe to which avidin is bound in advance is used, the binding probe is preferably purified before a hybridization step for forming the target substance detection polymer.

When the target substance detection polymer to which biotin is bound is used as the target substance detection polymer, a method of binding biotin to the binding probe is not particularly limited. For example, it is preferred that a probe having a region capable of binding to at least one of the nucleic acid probes for forming a polymer be labeled with biotin.
Although the time point at which biotin is bound to the binding probe is not limited, it is suitable to use the binding probe obtained by labeling biotin in advance and cause the binding probe and a nucleic acid probe to react with each other to form a target substance detection polymer to which biotin is bound.

FIG. 15 is a schematic explanatory view illustrating an example of the target substance detection polymer of the present invention, and illustrates an example in which the multiple kinds of nucleic acid probes illustrated in FIG. 1 are used. As illustrated in FIG. 15, a target substance detection polymer 52a having the target region T capable of binding to the target substance can be formed by causing the multiple kinds of nucleic acid probes 10 and 12 to react with a binding probe 50a in a solution.

The target substance detection polymer may be formed by causing the multiple kinds of nucleic acid probes and the binding probe to hybridize with one another simultaneously. Alternatively, the target substance detection polymer may be formed by causing the multiple kinds of nucleic acid probes to hybridize with each other to form a first polymer, and causing the first polymer and the binding probe to hybridize with each other.
A composition ratio between the nucleic acid probes and the binding probe in the target substance detection polymer is such that the amount of the binding probe falls within the range of preferably 0.1 to 20 parts by mol or more preferably 1 to 10 parts by mol with respect to 100 parts by mol of each nucleic acid probe.

According to the present invention, the size of the target substance detection polymer can be controlled depending on the concentration of each of the nucleic acid probes at the time of the polymer formation reaction. Increasing the concentration of each of the nucleic acid probes can provide a larger target substance detection polymer.
In the present invention, a condition for the concentration of each of the nucleic acid probes has only to be appropriately selected depending on a required size of the target substance detection polymer. The concentration of each nucleic acid probe is preferably set to 50 to 1000 pmol/mL, or more preferably 100 to 500 pmol/mL.
Although the concentration of the binding probe has only to be appropriately selected depending on the concentration of each of the nucleic acid probes, the concentration falls within the range of preferably 0.1 to 20 parts by mol or more preferably 1 to 10 parts by mol with respect to 100 parts by mol of each nucleic acid probe.

The composition and concentration of a reaction buffer are not particularly limited, and an ordinary buffer regularly used for nucleic acid amplification can be suitably used. The pH in a regular range is suitable, and a buffer having a pH in the range of 7.0 to 9.0 can be preferably used, provided that a pH in the range of 5.0 to 6.0 is preferred when a nucleic acid probe labeled with an acridinium ester is used. The temperature condition of the hybridization reaction is not particularly limited, and a usual temperature condition is appropriate. However, the temperature is preferably 40°C to 80°C, or more preferably 55°C to 65°C. Moreover, it is preferred that a reaction temperature region be partially formed in a reaction solution, and a self-assembly reaction be performed in the reaction temperature region (WO 2005/106031). The reaction temperature applied in the reaction temperature region partially formed is preferably 40 to 80°C, or more preferably 55 to 65°C.

Although the solution containing the target substance detection polymer may be used as it is after its production, the solution is preferably diluted to a proper concentration (for example, diluted 5 to 80 fold) as required. In the present invention, the target substance detection polymer is produced in advance. Accordingly, the target substance detection polymer of a predetermined size can be used upon detection of the target substance irrespective of the concentration of the target substance detection polymer. Reducing the concentration of the target substance detection polymer can suppress a non-specific reaction.

An unreacted nucleic acid probe is preferably removed from the resultant target substance detection polymer by, for example, gel filtration, ultrafiltration, or dialysis. In addition, the polymer is suitably fractionated into certain sizes, and a collection of the fractions is suitably used.

The target substance detection polymer of the present invention is preferably labeled with a labeling substance. Although a method for the labeling is not particularly limited, a nucleic acid probe labeled with the labeling substance in advance is preferably used. An acridinium ester, europium, ruthenium, terbium, Cy3, Alexa, a radioactive isotope, biotin, digoxigenin, a fluorescent substance, an emission substance, a pigment, or the like is suitably used as the labeling substance. Of those, the acridinium ester is particularly preferred in terms of operability, quantitativeness, and sensitivity.
Alternatively, a fluorescent substance having such property as to bind to a nucleic acid (for example, an intercalator such as ethidium bromide, an Oligreen, or an SYBR Green I) may be added to the formed polymer to label the target substance detection polymer.

The target substance detection polymer and the target substance are caused to react with each other so that the target substance detection polymer and the target substance are bound to each other. After that, the target substance detection polymer to which the target substance is bound is detected. Thus, the target substance is detected.

As a sample for measuring a target substance in the present invention, any sample having a possibility of containing the target substance can be applied. Examples of the sample include samples derived from living organisms such as blood, serum, urine, feces, cerebrospinal fluid, tissue fluid, sputum, and cell culture, and samples possibly containing or being infected by viruses, bacteria, molds, and the like.
Examples of the target substance include a nucleic acid, an antigen, an antibody, a receptor, a hapten, an enzyme, a protein, a peptide, a polymer, a carbohydrate, and a combination thereof. The target substance may be one suitably prepared from a sample or one isolated from a sample. Besides, nucleic acids such as DNA and RNA can also be used, the nucleic acids being obtained by amplifying a target nucleic acid in a sample by a known method. As the target nucleic acid (target gene), single-stranded DNA and/or RNA and double-stranded DNA and/or RNA can be used. In addition, SNPs (single nucleotide polymorphism) can be used as the target nucleic acid.

A method of detecting a target substance of the present invention preferably involves the use of a carrier on which a substance capable of capturing the target substance is immobilized. For example, when the target substance is a nucleic acid, it is preferred that a nucleic acid probe having a sequence complementary to that of a site different from a site to be bound to the binding probe be used as a capture probe, and a carrier having the capture probe immobilized on its surface be used. Constitution is suitably performed so that the binding probe is bound to the target nucleic acid in a state where the binding probe and the capture probe are adjacent to each other.
A substrate such as fluorescent fine particles, magnetic particles, a microplate, a microarray, a slide glass, or an electrically conductive substrate is preferably used as the carrier.

FIG. 16 is a schematic explanatory view illustrating another example of the target substance detection polymer of the present invention, and illustrates an example in which the multiple kinds of nucleic acid probes illustrated in FIG. 1 are used and a binding probe to which streptavidin is bound as avidin in advance is used. Although FIG. 16 illustrates an example of the binding probe to which avidin is bound, a binding probe to which biotin is bound instead of avidin can be similarly used.
As illustrated in FIG. 16, a target substance detection polymer 52b to which streptavidin S is bound can be formed by causing the multiple kinds of nucleic acid probes 10 and 12 to react with a binding probe 50b in a solution.

Although FIG. 16 illustrates an example in which a binding probe formed by binding avidin or biotin in advance (that is, a binding probe whose target region is avidin or biotin) is used, the target substance detection polymer may be formed by using a binding probe having a region to which avidin or biotin can be bound, causing the multiple kinds of nucleic acid probes for forming a polymer to hybridize with the binding probe to form a polymer, and binding avidin or biotin to the binding probe of the polymer.

After the target substance detection polymer and the target substance have been bound to each other through a bond between biotin and avidin, the target substance detection polymer to which the target substance is bound is detected. Thus, the target substance is detected.
When a target substance detection polymer to which avidin is bound is used as the target substance detection polymer, the target substance detection polymer and the target substance are bound to each other through biotin.
A method of binding the target substance and biotin is not particularly limited, and a known method can be employed. For example, when the target substance is a nucleic acid, a biotin-labeled probe having a region complementary to the target nucleic acid is preferably used. When the target substance is an antigen, a biotin-labeled antibody that specifically binds to the antigen is preferably used.

When a target substance detection polymer to which biotin is bound is used as the target substance detection polymer, the target substance detection polymer and the target substance are bound to each other through avidin.
A method of binding the target substance and avidin is not particularly limited, and a known method can be employed. For example, when the target substance is a nucleic acid, it is preferred that a biotin-labeled probe having a region complementary to the target nucleic acid be used to bind the target nucleic acid and biotin, and avidin be caused to react with the resultant so that the target nucleic acid and avidin are bound to each other. In addition, when the target substance is an antigen, it is preferred that a biotin-labeled antibody that specifically binds to the antigen be used to bind the antigen and biotin, and avidin be caused to react with the resultant so that the antigen and avidin are bound to each other. Alternatively, a probe having a region capable of binding to the target substance may be modified with, for example, NH₂, COOH, or SH, and avidin may be chemically bound to the resultant.

### Examples

Hereinafter, the present invention is described more specifically by way of examples, but these examples are illustrative only and are, of course, not for a restrictive construe.

### (Example 1)

A nucleic acid probe having the following base sequence (SEQ ID NO: 1) (hereinafter referred to as "HCP-1") and a nucleic acid probe having the following base sequence (SEQ ID NO: 2) (hereinafter referred to as "HCP-2") were used as multiple nucleic acid probes for polymer formation. The 5' terminal of each of the HCP-1 and the HCP-2 was labeled with an acridinium ester (AE).
Base sequence of HCP-1 (5'-X₁-X₂-X₃-3')
5'-GATGTCTCGGGATG GCTTCGGAGTTACG CTGGCGGTATCAAC-3' (SEQ ID NO: 1)
Base sequence of HCP-2 (5'-X₁'-X₂'-X₃'-3') 5'-CATCCCGAGACATC CGTAACTCCGAAGC GTTGATACCGCCAG-3' (SEQ ID NO: 2)

S.Aureus Oligo having the following base sequence (SEQ ID NO: 3) (hereinafter referred to as "target oligo") was used as a target substance.
Base sequence of target oligo 5'-TTCGGGAAACCGGAGCTAATA CCGGATAATATTTTGAACCGC ATGGTTCAAAAGTGAAAGACG GTCTTGCTGTCACTTATAGAT GGATCCGCGCTGCATTAGCTA-3' (SEQ ID NO: 3)

A nucleic acid probe having a sequence complementary to that of the target oligo (SEQ ID NO: 4) was used as a capture probe.
Base sequence of capture probe 5'-CGTCTTTCACTTTTGAACCAT GCGGTTCAAAATATTATCCGG-3' (SEQ ID NO: 4)

A nucleic acid probe having a base sequence complementary to that of the target oligo DNA and identical to part of that of the HCP-1 (SEQ ID NO: 5) was used as a binding probe.
Base sequence of binding probe (5'-X₁-X₂-X₁-T-3') 5'-GATGTCTCGGGATG GCTTCGGAGTTACG GATGTCTCGGGATG ATCTATAAGTGACAGCAAGAC-3' (SEQ ID NO: 5)

250 pmol/mL of each of the AE-labeled HCP-1 and the AE-labeled HCP-2 were added to a reaction liquid (100 mM of lithium succinate, 600 mM of lithium chloride, 2 mM of EDTA, 5% of LDS, an additive, pH 5.0). Then, 6.25 pmol/mL of the binding probe were added to the mixture. The resultant reaction liquid was subjected to a reaction while being stirred at 55°C for 10 minutes so that a polymer was formed. Thus, a polymer-containing solution was prepared.
The resultant polymer-containing solution was diluted with a reaction liquid (100 mM of lithium succinate, 600 mM of lithium chloride, 2 mM of EDTA, 5% of LDS, an additive, pH 5.0) 10 fold. Thus, a detection solution was prepared.

50 uL of 10 fmol/mL of the target oligo and 12.5 µL of a reaction solution (SSC 10X, 62.5 mM of Tris, pH 8.0) were added to the wells of a microplate (white) on which the capture probe had been immobilized. Then, an aluminum sheet to which filter paper had been stuck was stuck to the upper surface of the plate. After that, the plate was set in an incubator with its upper portion and lower portion set to 10°C and 65°C, respectively, and was then subjected to a reaction for 30 minutes.

After the reaction, the aluminum sheet was peeled, and then the wells of the microplate were washed by using a Washer (Model 1575 manufactured by Bio-Rad Laboratories, Inc.) with a washing liquid (50 mM of Tris, 300 mM of NaCl, 0.01% of Triton X-100, pH 7.0) five times. After the washing liquid had been completely decanted, 100 µL of the prepared detection solution were added to the wells. An aluminum sheet to which filter paper had been stuck was stuck to the upper surface of the plate. After that, the plate was set in an incubator with its upper portion and lower portion set to 10°C and 65°C, respectively, and was then subjected to a reaction for 10 minutes.

After the reaction, the aluminum sheet was peeled, and then the wells of the microplate were washed by using a Washer (Model 1575 manufactured by Bio-Rad Laboratories, Inc.) with the washing liquid five times. After the washing liquid had been completely decanted, 50 µL of each of an emission reagent I and an emission reagent II manufactured by Gen-Probe Incorporated were added to the wells with a luminometer Centro LB960 manufactured by BERTHOLD TECHNOLOGIES GmbH & Co. KG. Simultaneously with the addition, emission intensity was measured. Table 1 shows the result.

### (Example 2)

An oligo DNA was detected in the same manner as in Example 1 except that a method of preparing a detection solution was changed as described below. Table 1 shows the result.
250 pmol/mL of each of the AE-labeled HCP-1 and the AE-labeled HCP-2 were added to a reaction liquid (100 mM of lithium succinate, 600 mM of lithium chloride, 2 mM of EDTA, 5% of LDS, an additive, pH 5.0). After the mixture was reacted while being stirred at 55°C for 10 minutes, 6.25 pmol/mL of the binding probe were added to the resultant. The resultant reaction liquid was subjected to a reaction while being stirred at 55°C for 10 minutes so that a polymer was formed. Thus, a polymer-containing solution was prepared. Except the above-mentioned process, a detection solution was prepared in the same manner as in Example 1.

### (Comparative Example 1)

An oligo DNA was detected in the same manner as in Example 1 except that the reaction liquid (100 mM of lithium succinate, 600 mM of lithium chloride, 2 mM of EDTA, 5% of LDS, an additive, pH 5.0) containing 25 pmol/ml of each of the AE-labeled HCP-1 and the AE-labeled HCP-2, and 0.625 pmol/ml of the binding probe used in Example 1 was used instead of the detection solution. Table 1 shows the result.

**[Table 1]**

| | Relative emission intensity | Blank |
|---|---|---|
| Example 1 | 34,374 | 45 |
| Example 2 | 27,667 | 108 |
| Comparative Example 1 | 12,052 | 52 |

As shown in Table 1, Examples 1 and 2 each showed an emission intensity two to three times as high as that of Comparative Example 1.

### (Example 3)

After 250 pmol/mL of unlabeled HCP-1 (base sequence: SEQ ID NO: 1) and 275 pmol/mL of unlabeled HCP-2 (base sequence: SEQ ID NO: 2) were added to a reaction liquid (100 mM of lithium succinate, 600 mM of lithium chloride, 0.2% of Triton X-100, an additive, pH 7.5), 6.25 pmol/mL of a binding probe (base sequence: SEQ ID NO: 5) were added to the resultant. The resultant reaction liquid was reacted while being stirred at 55°C for 10 minutes to form a polymer. Thus, a polymer-containing solution was prepared.
The temperature of the resultant polymer-containing solution was returned to room temperature. An SYBR Green I (manufactured by CAMBREX Bio Science Rockland, Inc., trade name SYBR Green I Nucleic Acid) was added to the solution so that the final dilution ratio became 4000, and then the solution was stained at room temperature for 15 minutes. After that, the resultant was diluted with a reaction liquid (100 mM of lithium succinate, 600 mM of lithium chloride, 0.2% of Triton X-100, an additive, pH 7.5) five fold. Thus, a detection solution was prepared.

A nucleic acid probe having the following base sequence (SEQ ID NO: 6) (hereinafter referred to as "helper probe") was used as a target substance capture probe.
Base sequence of helper probe
5'-CGTCTTTCACTTTTGAACCAT
AAAAAAAAAAAAAAAAAAAAAAAAAAAAAA-3' (SEQ ID NO: 6)

After 200 µL of the prepared detection solution had been added to a polystyrene test tube, 20 pmol/mL of the helper probe and 25 µL (2000 amol) of the target oligo (SEQ ID NO: 3) were added to the test tube, and the mixture was subjected to a reaction in a water bath while being stirred at 55°C for 30 minutes. After the temperature of the resultant had been returned to room temperature, 2.5 µL of magnetic beads in which dT14 had been turned into a solid phase were added to the resultant, and then the mixture was subjected to a reaction at room temperature for 15 minutes.

The polystyrene test tube was taken out of the water bath, and was then brought into contact with a magnet. A washing liquid (20 mM of HEPES, 600 mM of LiCl, 1 mM of EDTA2Na, 0.05% of Triton X-100, pH 7.8) was added to the tube, and the suction and discharge of the washing liquid were repeated so that the magnetic beads were washed three times. Then, 100 µL of a TE buffer (20 mM of Tris, 1 mM of EDTA, pH 7.8) were added to the precipitate of the magnetic beads in the polystyrene test tube to refloat the magnetic beads. The total amount of the refloated magnetic beads were transferred to the wells of a microplate all of which were black, and the fluorescence intensity of the SYBR immobilized on the magnetic beads was measured with a fluorescent reader Berthold LB970 at an excitation wavelength of 485 nm and a fluorescent wavelength of 520 nm. Table 2 shows the result.

### (Example 4)

An oligo DNA was detected in the same manner as in Example 3 except that a method of preparing a detection solution was changed as described below. Table 2 shows the result.
The detection solution was prepared in the same manner as in Example 3 except that a polymer-containing solution was prepared by adding 250 pmol/mL of the unlabeled HCP-1 and 275 pmol/mL of the unlabeled HCP-2 to a reaction liquid (100 mM of lithium succinate, 600 mM of lithium chloride, 0.2% of Triton X-100, an additive, pH 7.5), subjecting the mixture to a reaction while stirring the mixture at 55°C for 10 minutes, adding 6.25 pmol/mL of the binding probe to the resultant, and subjecting the mixture to a reaction while stirring the mixture at 55°C for 10 minutes to form a polymer.

### (Comparative Example 2)

An oligo DNA was detected in the same manner as in Example 3 except that the reaction liquid (100 mM of lithium succinate, 600 mM of lithium chloride, 0.2% of Triton X-100, an additive, pH 7.5) containing 250 pmol/mL of the unlabeled HCP-1, 275 pmol/mL of the unlabeled HCP-2, and 1.25 pmol/mL of the binding probe used in Example 3 was used instead of the detection solution. Table 2 shows the result.

**[Table 2]**

| | Fluorescence intensity (after blank correction) |
|---|---|
| Example 3 | 722 |
| Example 4 | 525 |
| Comparative Example 2 | 329 |

As shown in Table 2, Examples 3 and 4 each showed fluorescence intensity one and a half to two times as high as that of Comparative Example 2.

### (Example 5)

A nucleic acid probe having the following base sequence (SEQ ID NO: 7) (hereinafter referred to as "dimer probe-1"), a nucleic acid probe having the following base sequence (SEQ ID NO: 8) (hereinafter referred to as "dimer probe-2"), a nucleic acid probe having the following base sequence (SEQ ID NO: 9) (hereinafter referred to as "dimer probe-3"), and a nucleic acid probe having the following base sequence (SEQ ID NO: 10) (hereinafter referred to as "dimer probe-4") were used as multiple nucleic acid probes for polymer formation. The 5' terminal of each of the dimer probes-1 to 4 was labeled with an acridinium ester (AE).
Base sequence of dimer probe-1
5'-CATCTCTGCTGGTC CCTCGGCTGCGTCG GTTCGCCATAGACG-3' (SEQ ID NO: 7)
Base sequence of dimer probe-2
5'-GCACATTCACACCG CGACGCAGCCGAGG CCTGACCTCTATGC-3' (SEQ ID NO: 8)
Base sequence of dimer probe-3 5'-GACCAGCAGAGATG GCAGCGACGGCACC CGTCTATGGCGAAC-3' (SEQ ID NO: 9)
Base sequence of dimer probe-4
5'-CGGTGTGAATGTGC GGTGCCGTCGCTGC GCATAGAGGTCAGG-3' (SEQ ID NO: 10)

A nucleic acid probe having a base sequence complementary to that of a target oligo DNA and identical to the entirety of that of the dimer probe-1 (SEQ ID NO: 11) was used as a binding probe.
Base sequence of binding probe 5'-CATCTCTGCTGGTC CCTCGGCTGCGTCG GTTCGCCATAGACG ATCTATAAGTGACAGCAAGAC-3' (SEQ ID NO: 11)

250 pmol/mL of each of the AE-labeled dimer probe-1 and the AE-labeled dimer probe-2 were added to and mixed in a reaction liquid (100 mM of lithium succinate, 0.3 M of lithium chloride, 2 mM of EDTA2Na, 2 mM of EGTA, 5% of lithium dodecylsulfate (LDS), an additive, pH 5.0). Thus, a first dimer probe solution was obtained. In addition, 250 pmol/mL of each of the AE-labeled dimer probe-3 and the AE-labeled dimer probe-4 were added to and mixed in the reaction liquid. Thus, a second dimer probe solution was obtained.
Equal amounts of the first dimer probe solution and the second dimer probe solution were added to a test tube into which a condensate of the binding probe (SEQ ID NO: 11) had been incorporated in advance (it should be noted that, in this case, the concentration of each of the dimer probes-1 to 4 was 125 pmol/mL and the concentration of the binding probe was 6.25 pmol/mL). After that, the mixture was subjected to a reaction while being left at rest at 60°C for 10 minutes so that a polymer was formed. Thus, a polymer-containing solution was prepared.
The resultant polymer-containing solution was diluted with a reaction liquid (100 mM of lithium succinate, 600 mM of lithium chloride, 2 mM of EDTA2Na, 2 mM of EGTA, 5% of lithium dodecylsulfate (LDS), an additive, pH 5.0) 40 fold. Thus, a detection solution was prepared.

After 200 µL of the prepared detection solution had been added to a polystyrene test tube, 20 pmol/mL of the helper probe (SEQ ID NO: 6) and 25 µL (100 amol/tube) of the target oligo (SEQ ID NO: 3) were added to the test tube, and the mixture was subjected to a reaction in a water bath while being stirred at 55°C for 30 minutes. After the temperature of the resultant had been returned to room temperature, 2.5 µL of magnetic beads in which dT14 had been turned into a solid phase were added to the resultant, and then the mixture was subjected to a reaction at room temperature for 15 minutes.

The polystyrene test tube was taken out of the water bath, and was then brought into contact with a magnet. A washing liquid (20 mM of PIPES, 100 mM of LiCl, 1 mM of EDTA2Na, 0.05% of lithium dodecylsulfate (LDS), pH 6.0) was added to the tube, and the suction and discharge of the washing liquid were repeated so that the magnetic beads were washed three times. Then, 100 µL of a washing liquid (20 mM of PIPES, 100 mM of LiCl, 1 mM of EDTA2Na, 0.05% of lithium dodecylsulfate (LDS), pH 6.0) were added to the precipitate of the magnetic beads in the polystyrene test tube to refloat the magnetic beads. The total amount of the refloated magnetic beads was transferred to a new polystyrene test tube. The test tube was set to a luminometer (Berthold Lumat LB 9507), and 200 µL of each of the emission reagent I and the emission reagent II manufactured by Gen-Probe Incorporated were added to measure the emission quantity. Table 3 shows the result.

### (Comparative Example 3)

6.25 pmol/mL of each of the AE-labeled dimer probes-1 and 2 (SEQ ID NOS: 7 and 8) were added to and mixed in a reaction liquid (100 mM of lithium succinate, 600 mM of lithium chloride, 2 mM of EDTA2Na, 2 mM of EGTA, 5% of lithium dodecylsulfate (LDS), an additive, pH 5.0). Thus, a third dimer probe solution was obtained. Next, 6.25 pmol/mL of each of the AE-labeled dimer probes-3 and 4 (SEQ ID NOS: 9 and 10) were added to and mixed in the reaction liquid. Thus, a fourth dimer probe solution was obtained.
Equal amounts of the third dimer probe solution and the fourth dimer probe solution were mixed with each other, and then the binding probe (SEQ ID NO: 11) was added to the mixture so as to have a concentration of 0.156 pmol/mL. Thus, a detection solution was prepared.

The target oligo was detected in the same manner as in Example 5 except that the resultant detection solution was used. Table 3 shows the result.

**[Table 3]**

| | Fluorescence intensity (RLU) (after blank correction) |
|---|---|
| Example 5 | 116,569 |
| Comparative Example 3 | 7,401 |

As shown in Table 3, Example 5 showed an emission intensity 16 times as high as that of Comparative Example 3.

### (Example 6)

A target oligo was detected in the same manner as in Example 5 except that the concentration of the target oligo to be added was changed to 0, 0.5, 1, 2, 5, 10, and 50 amol/tube. FIG. 15 illustrates the results. As illustrated in FIG. 15, good linearity passing through the origin was obtained.

### (Example 7)

The dimer probe-1 (SEQ ID NO: 7), the dimer probe-2 (SEQ ID NO: 8), a nucleic acid probe having the following base sequence (SEQ ID NO: 12) (hereinafter referred to as "crosslinking probe-1"), and a nucleic acid probe having the following base sequence (SEQ ID NO: 13) (hereinafter referred to as "crosslinking probe-2") were used as multiple nucleic acid probes for polymer formation. The 5' terminal of each of the dimer probes-1 and 2 was labeled with an acridinium ester (AE).
Base sequence of crosslinking probe-1 5'-GACCAGCAGAGATG CGTCTATGGCGAAC-3' (SEQ ID NO: 12)
Base sequence of crosslinking probe-2
5'-CGGTGTGAATGTGC GCATAGAGGTCAGG-3' (SEQ ID NO: 13)

250 pmol/mL of each of the AE-labeled dimer probes-1 and 2 were added to and mixed in a reaction liquid (100 mM of lithium succinate, 0.3 M of lithium chloride, 2 mM of EDTA2Na, 2 mM of EGTA, 5% of lithium dodecylsulfate (LDS), an additive, pH 5.0). Further, 250 pmol/mL of each of the crosslinking probes-1 and 2 were added to the mixture. Thus, a reaction solution was obtained.
The reaction solution was added to a test tube into which a condensate of the binding probe (SEQ ID NO: 11) had been incorporated in advance (it should be noted that, in this case, the concentration of each of the dimer probes-1 and 2 was 250 pmol/mL, the concentration of each of the crosslinking probes-1 and 2 was 250 pmol/mL, and the concentration of the binding probe was 18.75 pmol/mL). After that, the mixture was subjected to a reaction while being left at rest at 60°C for 10 minutes so that a polymer was formed. Thus, a polymer-containing solution was prepared.
The resultant polymer-containing solution was diluted with a reaction liquid (100 mM of lithium succinate, 600 mM of lithium chloride, 2 mM of EDTA2Na, 2 mM of EGTA, 5% of lithium dodecylsulfate (LDS), an additive, pH 5.0) 40 fold. Thus, a detection solution was prepared.

The target oligo was detected in the same manner as in Example 5 except that the resultant detection solution was used. Table 4 shows the result.

### (Comparative example 4)

6.25 pmol/mL of each of the AE-labeled dimer probes-1 and 2 were added to and mixed in a reaction liquid (100 mM of lithium succinate, 600 mM of lithium chloride, 2 mM of EDTA2Na, 2 mM of EGTA, 5% of lithium dodecylsulfate (LDS), an additive, pH 5.0). Further, 6.25 pmol/mL of each of the crosslinking probes-1 and 2 and 0.489 pmol/ml of the binding probe (SEQ ID NO: 11) were added to the mixture. Thus, a reaction solution was obtained.

The target oligo was detected in the same manner as in Example 7 except that the resultant detection solution was used. Table 4 shows the result.

**[Table 4]**

| | Fluorescence intensity (RLU) (after blank correction) |
|---|---|
| Example 7 | 165,069 |
| Comparative Example 4 | 13,674 |

As shown in Table 4, Example 7 showed an emission intensity 12 times as high as that of Comparative Example 4.

### (Example 8)

The AE-labeled HCP-1 (SEQ ID NO: 1) and the AE-labeled HCP-2 (SEQ ID NO: 2) used in Example 1 were used as multiple nucleic acid probes for polymer formation.
A nucleic acid probe which had the same base sequence as that of the HCP-1 and to which biotin was bound (SEQ ID NO: 14) was used as a binding probe.
Base sequence of binding probe (5'-polyT-X₁-X₂-X₃-3') 5'-Biotin-TTTTTTTTTT GATGTCTCGGGATG GCTTCGGAGTTACG CTGGCGGTATCAAC-3' (SEQ ID NO: 14)

10 pmol/mL of streptavidin and 10 pmol/mL of the binding probe were added to 0.5 ml of a reaction liquid (100 mM of lithium succinate, 600 mM of lithium chloride, 2 mM of EDTA, an additive, pH 5.0). Then, the mixture was subjected to a reaction while being stirred at room temperature for 30 minutes. Thus, a probe solution containing the binding probe to which streptavidin was bound was prepared.

250 pmol/mL of each of the AE-labeled HCP-1 and the AE-labeled HCP-2 were added to the prepared probe solution, and then the mixture was heated at 60°C for 30 minutes so that a polymer was formed. Thus, a polymer-containing solution was prepared.
The resultant polymer-containing solution was diluted with a reaction liquid (100 mM of lithium succinate, 600 mM of lithium chloride, 2 mM of EDTA, an additive, pH 7.0) 10 fold. Thus, a detection solution was prepared.

The following measurement was performed with a measuring kit for a sialylated carbohydrate antigen KL-6 (manufactured by Sanko Junyaku Co., Ltd., trade name: Eitest (registered trademark of Eisai Co., Ltd.) KL-6) in conformity with an operation manual included with the kit.
100 µL of a 20 mM Tris buffer (pH 7.5) to which 5 µg/mL of a KL-6 monoclonal antibody had been added were added to the wells of a white microplate so that the antibody were immobilized on the bottom surface of the plate. After the resultant had been washed with a washing liquid (20 mM of Tris, 150 mM of NaCl, 0.5% of Tween 20), 350 µL of a Tris buffer (pH 7.5) containing 1% of BSA were added to the resultant, and then the mixture was left at rest at 4°C for 3 hours. After that, the content liquid was disposed of. Thus, a KL-6 monoclonal antibody solid phase plate was produced.

100 µL of a reaction solution included with the kit were dispensed into the wells of the produced KL-6 monoclonal antibody solid phase plate, and then 20 µL of a standard antigen (0, 1, 2.5, 5, 10, or 20 U/mL) were dispensed. After that, the resultant was subjected to a reaction at room temperature for 2 hours.
After the reaction, each well was washed with a washing liquid I included with the kit three times. After that, 100 µL of a PBST buffer containing 1 µg/mL of a biotin-labeled KL-6 antibody (Sigma P3563, containing 2% of rabbit serum) were dispensed, and then the resultant was subjected to a reaction at room temperature for 1 hour.

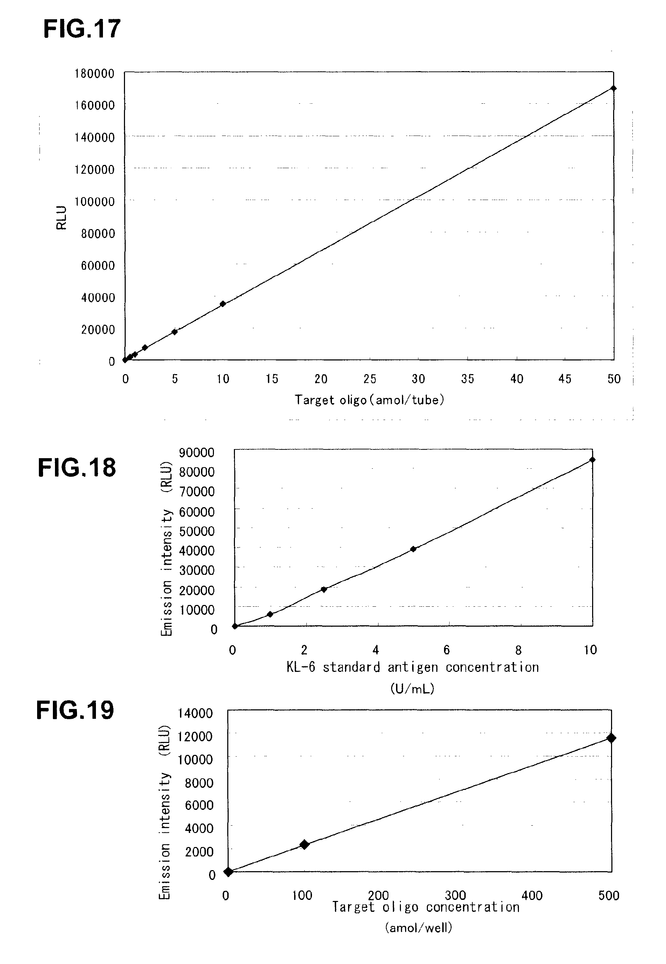

After the reaction, each well was washed with the washing liquid I three times. After that, 100 µL of the prepared detection solution were dispensed, and then the resultant was subjected to a reaction at room temperature for 30 minutes while the microplate was shaken. After the reaction, each well was washed with a washing liquid II (50 mM of Tris, 300 mM of NaCl, 0.01% of Triton X-100, 0.01% of NaN₃, pH 7.0) three times.
After the washing, 50 µL of each of the emission reagent I and the emission reagent II manufactured by Gen-Probe Incorporated were added, and the emission quantity of the AE was measured with an LB-960 manufactured by BERTHOLD TECHNOLOGIES GmbH & Co KG. FIG. 18 illustrates the results. As illustrated in FIG. 18, the present invention enabled high-sensitivity, quantitative measurement for the antigen.

### (Example 9)

The AE-labeled HCP-1 (SEQ ID NO: 1) and the AE-labeled HCP-2 (SEQ ID NO: 2) used in Example 1 were used as multiple nucleic acid probes for polymer formation.
A nucleic acid probe which had the same base sequence as that of the HCP-1 and to which biotin was bound (SEQ ID NO: 15) was used as a binding probe.
Base sequence of binding probe (5'-polyC-X₁X₂-X₃-3') 5'-Biotin-CCCCCCCCCC GATGTCTCGGGATG GCTTCGGAGTTACG CTGGCGGTATCAA-3' (SEQ ID NO: 15)

10 pmol/mL of streptavidin and 10 pmol/mL of the binding probe were added to 0.5 ml of a reaction liquid (100 mM of lithium succinate, 600 mM of lithium chloride, 2 mM of EDTA, an additive, pH 5.0). Then, the mixture was subjected to a reaction while being stirred at room temperature for 30 minutes. Thus, a probe solution containing the binding probe to which streptavidin was bound was prepared.

250 pmol/mL of each of the AE-labeled HCP-1 and the AE-labeled HCP-2 were added to the prepared probe solution, and then the mixture was heated at 60°C for 30 minutes so that a polymer was formed. Thus, a polymer-containing solution was prepared.
The resultant polymer-containing solution was diluted with a reaction liquid (100 mM of lithium succinate, 600 mM of lithium chloride, 2 mM of EDTA, an additive, pH 7.0) 20 fold. Thus, a detection solution was prepared.

The target oligo (SEQ ID NO: 3) used in Example 1 was used as a target substance.
The helper probe (SEQ ID NO: 6) used in Example 3 was used as a target substance capture probe.
A biotin-labeled nucleic acid probe having a base sequence complementary to that of the target oligo (SEQ ID NO: 16, hereinafter referred to as "biotin-labeled probe") was used as a binding probe the target substance detection polymer and the target substance.
Base sequence of biotin-labeled probe 5'-Biotin-CCCCCCCC CCATCTATAAGTGACAGCAAG-3' (SEQ ID NO: 16)

A nucleic acid probe having a sequence complementary to that of the helper probe (SEQ ID NO: 17, hereinafter referred to as "second capture probe") was immobilized on the wells of a white microplate. Thus, a microplate was prepared.
Base sequence of second capture probe 5'-NH₂-TTTTTTTTTTTTTTTTTTTTT-3' (SEQ ID NO: 17)

95 µL of a reaction liquid (100 mM of lithium succinate, 600 mM of lithium chloride, 2 mM of EDTA, an additive, pH 7.0) containing 5 pmol/mL of the helper probe and 0.25 pmol/mL of the biotin-labeled probe and 25 µL of the target oligo (target oligo concentration; 0, 100, or 500 amol/well) were added to a test tube, and then the mixture was subjected to a reaction at 55°C for 30 minutes. After that, 5 µL of the prepared detection solution were added to the resultant, and then the mixture was subjected to a reaction at room temperature for 30 minutes.

Next, the total amount of the content liquid in the test tube was transferred to the wells of the prepared microplate, and then the resultant was subjected to a reaction at room temperature for 30 minutes while the microplate was shaken.
After the wells of the microplate had been washed with a washing liquid five times, 50 µL of each of the emission reagent I and the emission reagent II manufactured by Gen-Probe Incorporated were added, and the emission quantity of the AE was measured with an LB-960 manufactured by BERTHOLD TECHNOLOGIES GmbH & Co KG. FIG. 19 illustrates the results. As illustrated in FIG. 19, good linearity passing through the origin was obtained, and the present invention enabled high-sensitivity, quantitative measurement for the target oligo.

## Claims

1. A method of detecting a target substance, comprising the steps of:
(A) forming a target substance detection polymer by causing multiple kinds of nucleic acid probes for forming a polymer to react with a binding probe comprising a target region capable of directly or indirectly binding to the target substance and a region capable of binding to at least one of the nucleic acid probes in a solution;
(B) binding the target substance to the target substance detection polymer; and
(C) detecting the target substance detection polymer to which the target substance is bound,
wherein the multiple kinds of nucleic acid probes comprise
a nucleic acid probe comprising at least n (n≥3) nucleic acid regions formed of a nucleic acid region X₁, a nucleic acid region X₂, ···, and a nucleic acid region Xₙ from a 5' terminal side in the stated order, and
a nucleic acid probe comprising at least n (n≥3) nucleic acid regions formed of a nucleic acid region X₁' complementary to the nucleic acid region X₁, a nucleic acid region X₂' complementary to the nucleic acid region X₂, ···, and a nucleic acid region Xₙ' complementary to the nucleic acid region Xₙ from a 5' terminal side in the stated order.

2. A method of detecting a target substance, comprising the steps of:
(A) forming a target substance detection polymer by causing multiple kinds of nucleic acid probes for forming a polymer to react with a binding probe comprising a target region capable of directly or indirectly binding to the target substance and a region capable of binding to at least one of the nucleic acid probes in a solution;
(B) binding the target substance to the target substance detection polymer; and
(C) detecting the target substance detection polymer to which the target substance is bound,
wherein the multiple kinds of nucleic acid probes each comprise a base sequence that hybridizes with any other kind of nucleic acid probe as represented by the following formula (I): 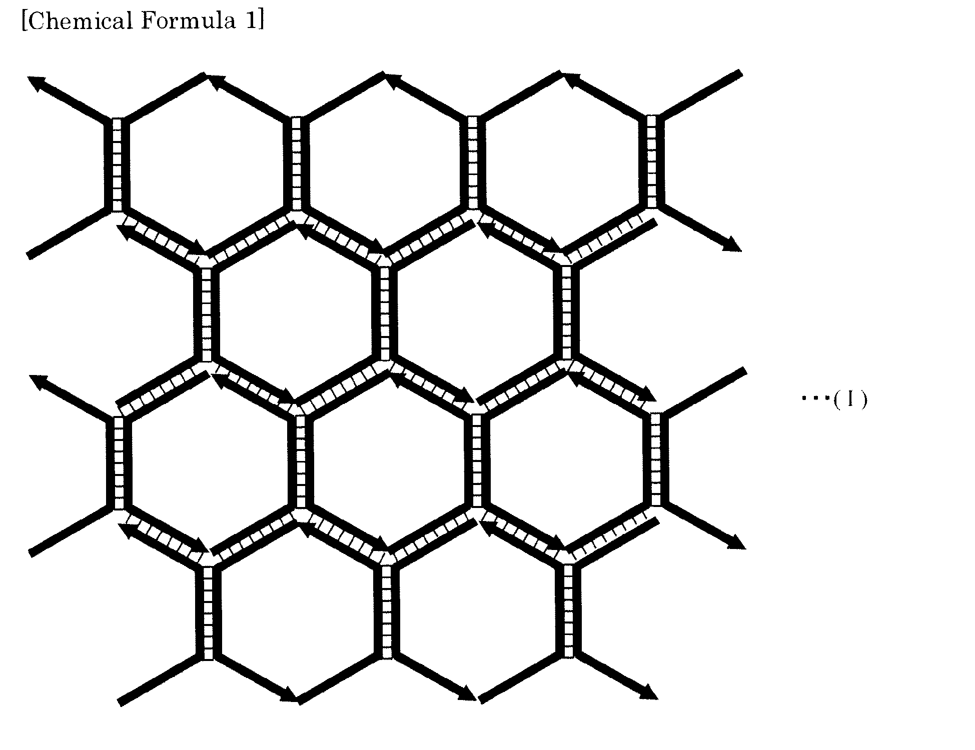 wherein two straight lines illustrated in a ladder fashion represent nucleic acids that hybridize with each other.

3. A method of detecting a target substance, comprising the steps of:
(A) forming a target substance detection polymer by causing multiple kinds of nucleic acid probes for forming a polymer to react with a binding probe comprising a target region capable of directly or indirectly binding to the target substance and a region capable of binding to at least one of the nucleic acid probes in a solution;
(B) binding the target substance to the target substance detection polymer; and
(C) detecting the target substance detection polymer to which the target substance is bound,
wherein the multiple kinds of nucleic acid probes each comprise a base sequence that hybridizes with any other kind of nucleic acid probe as represented by the following formula (II): 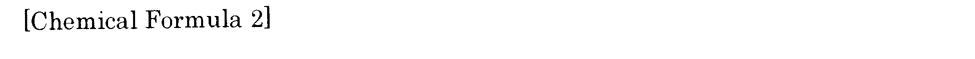 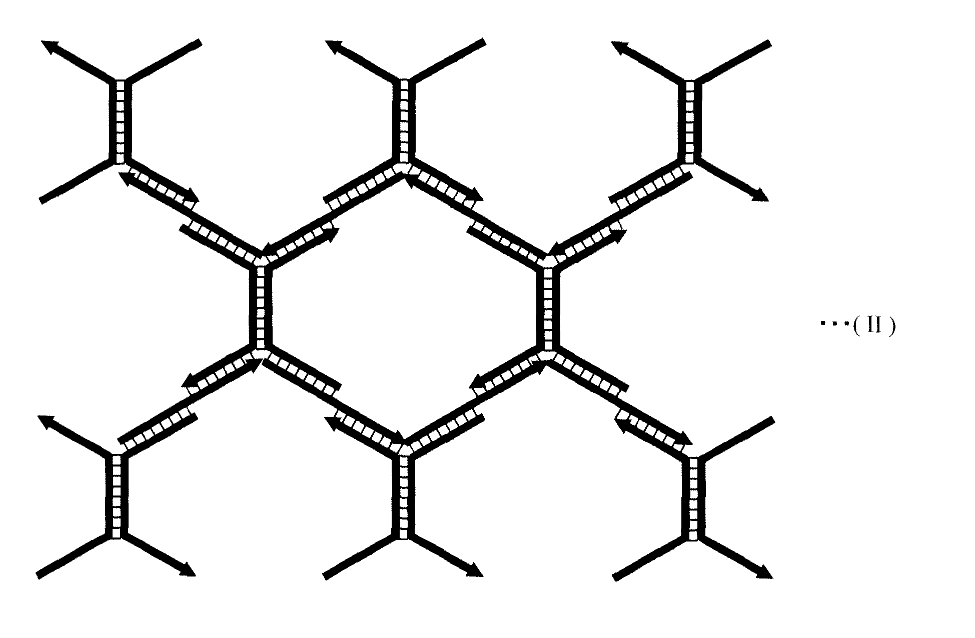 wherein two straight lines illustrated in a ladder fashion represent nucleic acids that hybridize with each other.

4. A method of detecting a target substance according to any one of claims 1 to 3, wherein the formation step comprises
a first hybridization step of causing the multiple kinds of nucleic acid probes to hybridize with each other to form a first polymer and
a second hybridization step of causing the first polymer and the binding probe to hybridize with each other to form the target substance detection polymer.

5. A method of detecting a target substance according to any one of claims 1 to 3, wherein the formation step comprises the step of causing the multiple kinds of nucleic acid probes and the binding probe to hybridize with one another simultaneously.

6. A method of detecting a target substance according to any one of claims 1 to 5, further comprising the step of diluting a solution containing the target substance detection polymer after the formation step to prepare a detection solution.

7. A method of detecting a target substance according to any one of claims 1 to 6, wherein at least one kind of nucleic acid probe of the multiple kinds of nucleic acid probes is labeled with a labeling substance.

8. A method of detecting a target substance according to any one of claims 1 to 7, wherein the target region of the binding probe comprises a portion capable of specifically binding to the target substance.

9. A method of detecting a target substance according to claim 8, wherein:
the target substance comprises a nucleic acid; and
the target region of the binding probe comprises a nucleic acid comprising a base sequence complementary to that of the target nucleic acid.

10. A method of detecting a target substance according to any one of claims 1 to 7, wherein the target substance detection polymer and the target substance are bound to each other through a spacer substance comprising a region capable of binding to the target substance and a region capable of binding to the target region of the binding probe.

11. A method of detecting a target substance according to any one of claims 1 to 8 and 10, wherein the target substance detection polymer is bound to avidin or biotin through the binding probe.

12. A method of detecting a target substance according to claim 11, wherein the binding step comprises the step of binding the target substance to the target substance detection polymer through a bond between biotin and avidin.

13. A method of forming a target substance detection polymer, the method comprising the step of forming a target substance detection polymer by causing multiple kinds of nucleic acid probes for forming a polymer to hybridize with a binding probe comprising a target region capable of directly or indirectly binding to the target substance and a region capable of binding to at least one of the nucleic acid probes,
wherein:
the binding probe is free of being bound to the target substance; and
the multiple kinds of nucleic acid probes comprise
a nucleic acid probe comprising at least n (n≥3) nucleic acid regions formed of a nucleic acid region X₁, a nucleic acid region X₂, ···, and a nucleic acid region Xₙ from a 5' terminal side in the stated order, and
a nucleic acid probe comprising at least n (n≥3) nucleic acid regions formed of a nucleic acid region X₁' complementary to the nucleic acid region X₁, a nucleic acid region X₂' complementary to the nucleic acid region X₂, ···, and a nucleic acid region Xₙ' complementary to the nucleic acid region Xₙ from a 5' terminal side in the stated order.

14. A method of forming a target substance detection polymer, the method comprising the step of forming the target substance detection polymer by causing multiple kinds of nucleic acid probes for forming a polymer to hybridize with a binding probe comprising a target region capable of directly or indirectly binding to the target substance and a region capable of binding to at least one of the nucleic acid probes,
wherein:
the binding probe is free of being bound to the target substance; and
the multiple kinds of nucleic acid probes each comprise a base sequence that hybridizes with any other kind of nucleic acid probe as represented by the following formula (I): 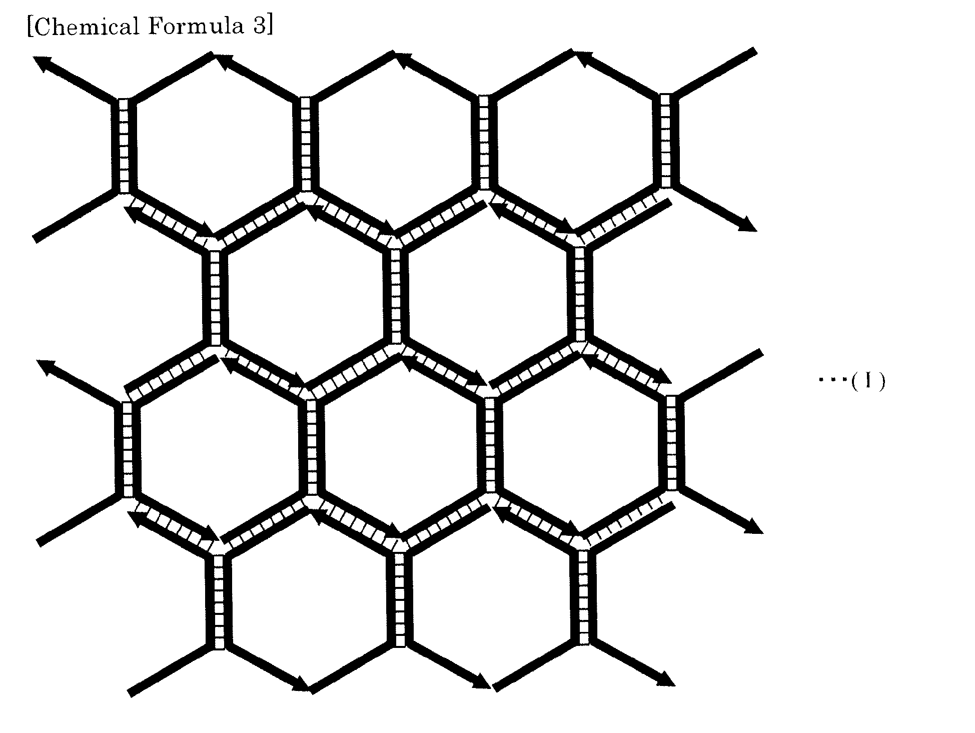 wherein two straight lines illustrated in a ladder fashion represent nucleic acids that hybridize with each other.

15. A method of forming a target substance detection polymer, the method comprising the step of forming the target substance detection polymer by causing multiple kinds of nucleic acid probes for forming a polymer to hybridize with a binding probe comprising a target region capable of directly or indirectly binding to the target substance and a region capable of binding to at least one of the nucleic acid probes,
wherein:
the binding probe is free of being bound to the target substance; and
the multiple kinds of nucleic acid probes each comprise a base sequence that hybridizes with any other kind of nucleic acid probe as represented by the following formula (II): 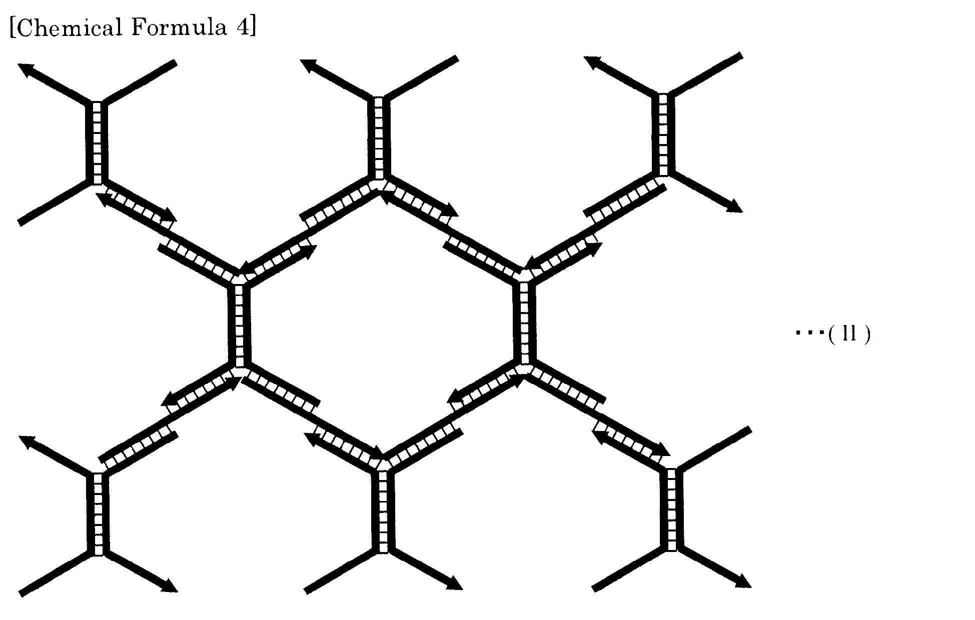 wherein two straight lines illustrated in a ladder fashion represent nucleic acids that hybridize with each other.

16. A method of forming a target substance detection polymer according to any one of claims 13 to 15, wherein the formation step comprises
a first hybridization step of causing the multiple kinds of nucleic acid probes to hybridize with each other to form a first polymer and
a second hybridization step of causing the first polymer and the binding probe to hybridize with each other to form the target substance detection polymer.

17. A method of forming a target substance detection polymer according to any one of claims 13 to 15, wherein the formation step comprises the step of causing the multiple kinds of nucleic acid probes and the binding probe to hybridize with one another simultaneously.

18. A method of forming a target substance detection polymer according to any one of claims 13 to 17, wherein at least one kind of nucleic acid probe of the multiple kinds of nucleic acid probes is labeled with a labeling substance.

19. A method of forming a target substance detection polymer according to any one of claims 13 to 18, wherein the target region of the binding probe comprises a portion capable of specifically binding to the target substance.

20. A method of forming a target substance detection polymer according to claim 19, wherein:
the target substance comprises a nucleic acid; and
the target region of the binding probe comprises a nucleic acid comprising a base sequence complementary to that of the target nucleic acid.

21. A method of forming a target substance detection polymer according to any one of claims 13 to 19, wherein the target substance detection polymer is bound to avidin or biotin through the binding probe.

22. A method of forming a target substance detection polymer according to claim 21, wherein the target region of the binding probe is bound to avidin or biotin.

23. A method of forming a target substance detection polymer according to claim 21, wherein:
the target region of the binding probe comprises a region which is capable of directly or indirectly binding avidin or biotin; and
the formation step comprises the steps of
forming a second polymer by causing the multiple kinds of nucleic acid probes for forming a polymer to hybridize with the binding probe and
binding avidin or biotin to the binding probe of the second polymer.

24. A target substance detection polymer, comprising:
multiple kinds of nucleic acid probes for forming a polymer; and
a binding probe comprising a target region capable of directly or indirectly binding to the target substance and a region capable of binding to at least one of the nucleic acid probes,
the target substance detection polymer being formed by causing the multiple kinds of nucleic acid probes to hybridize with the binding probe and the binding probe being free of being bound to the target substance,
wherein the multiple kinds of nucleic acid probes comprise
a nucleic acid probe comprising at least n (n≥3) nucleic acid regions formed of a nucleic acid region X₁, a nucleic acid region X₂, ···, and a nucleic acid region Xₙ from a 5' terminal side in the stated order, and
a nucleic acid probe comprising at least n (n≥3) nucleic acid regions formed of a nucleic acid region X₁' complementary to the nucleic acid region X₁, a nucleic acid region X₂' complementary to the nucleic acid region X₂, ···, and a nucleic acid region Xₙ' complementary to the nucleic acid region Xₙ from a 5' terminal side in the stated order.

25. A target substance detection polymer, comprising:
multiple kinds of nucleic acid probes for forming a polymer; and
a binding probe comprising a target region capable of directly or indirectly binding to the target substance and a region capable of binding to at least one of the nucleic acid probes,
the target substance detection polymer being formed by causing the multiple kinds of nucleic acid probes to hybridize with the binding probe and the binding probe being free of being bound to the target substance,
wherein the multiple kinds of nucleic acid probes each comprise a base sequence that hybridizes with any other kind of nucleic acid probe as represented by the following formula (I): 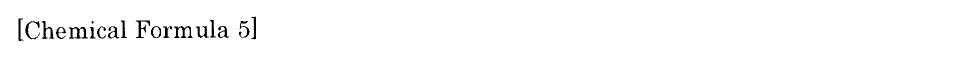 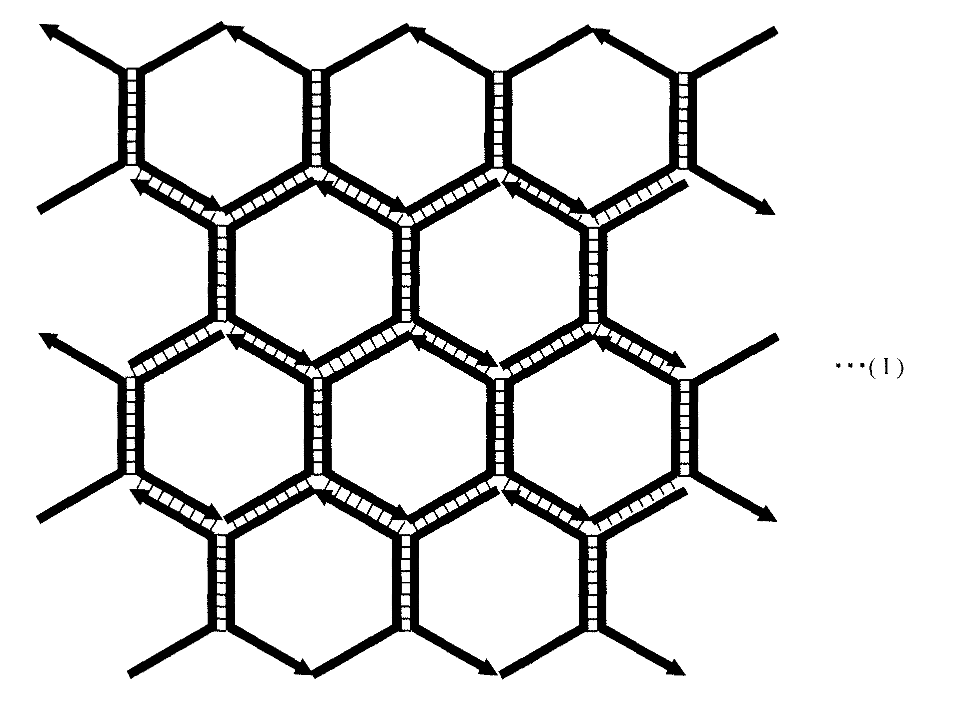 wherein two straight lines illustrated in a ladder fashion represent nucleic acids that hybridize with each other.

26. A target substance detection polymer, comprising:
multiple kinds of nucleic acid probes for forming a polymer; and
a binding probe comprising a target region capable of directly or indirectly binding to the target substance and a region capable of binding to at least one of the nucleic acid probes,
the target substance detection polymer being formed by causing the multiple kinds of nucleic acid probes to hybridize with the binding probe and the binding probe being free of being bound to the target substance,
wherein the multiple kinds of nucleic acid probes each comprise a base sequence that hybridizes with any other kind of nucleic acid probe as represented by the following formula (II): 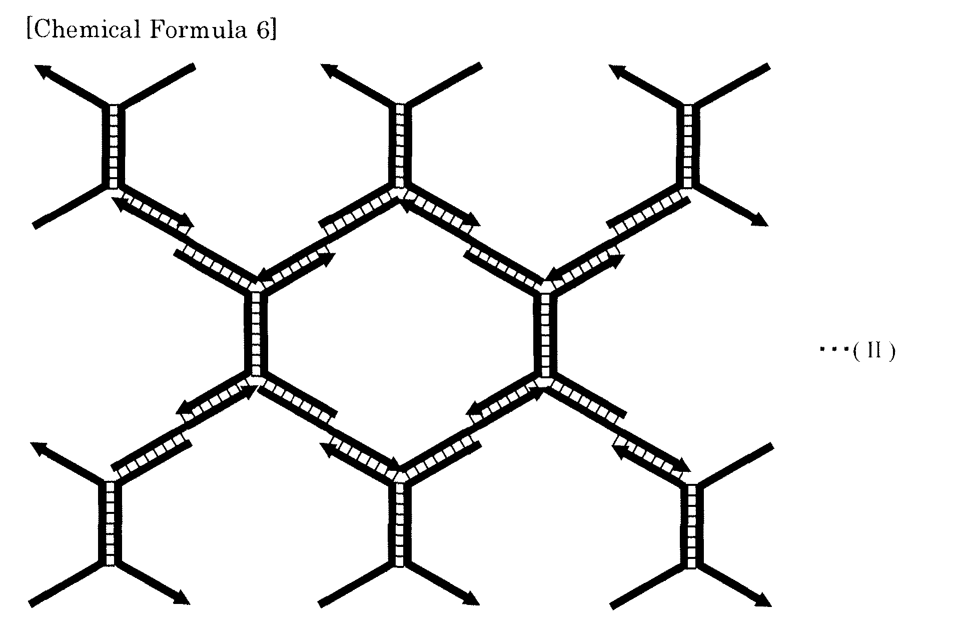 wherein two straight lines illustrated in a ladder fashion represent nucleic acids that hybridize with each other.

27. A target substance detection polymer according to any one of claims 24 to 26, wherein the target region of the binding probe comprises a portion capable of specifically binding to the target substance.

28. A target substance detection polymer according to claim 27, wherein:
the target substance comprises a nucleic acid; and
the target region of the binding probe comprises a nucleic acid comprising a base sequence complementary to that of the target nucleic acid.

29. A target substance detection polymer according to any one of claims 24 to 27, wherein the target substance detection polymer is bound to avidin or biotin through the binding probe.

30. A target substance detection polymer formed by the method according to any one of claims 13 to 23.

31. A method of detecting a target substance, comprising the steps of:
binding the target substance to the target substance detection polymer according to any one of claims 24 to 30; and
detecting the target substance detection polymer to which the target substance is bound.

32. A method of detecting a target substance according to claim 31, wherein the target substance detection polymer and the target substance are bound to each other through a spacer substance comprising a region capable of binding to the target substance and a region capable of binding to the target region of the binding probe.

33. A method of detecting a target substance according to claim 31, wherein the binding step comprises the step of binding the target substance to the target substance detection polymer according to claim 29 through a bond between biotin and avidin.


SEQUENCE LISTING

<110>  Eisai R&D Management Co., Ltd.

<120>  POLYMER FOR DETECTION OF TARGET SUBSTANCE, AND
       METHOD FOR DETECTION OF TARGET SUBSTANCE

<130>  EP70359FZpau

<140>  08 844 837.8
<141>  2008-10-30

<150>  PCT/JP2008/069778
<151>  2008-10-30

<150>  JP 2007-283672
<151>  2007-10-31

<150>  JP 2008-153974
<151>  2008-06-12

<160>  17

<170>  PatentIn version 3.1

<210>  1
<211>  42
<212>  DNA
<213>  Artificial

<220>
<223>  Description of Artificial Sequence; Synthetic

<400>  1
gatgtctcgg gatggcttcg gagttacgct ggcggtatca ac                    42


<210>  2
<211>  42
<212>  DNA
<213>  Artificial

<220>
<223>  Description of Artificial Sequence; Synthetic

<400>  2
catcccgaga catccgtaac tccgaagcgt tgataccgcc ag                    42


<210>  3
<211>  105
<212>  DNA
<213>  Artificial

<220>
<223>  Description of Artificial Sequence; Synthetic

<400>  3
ttcgggaaac cggagctaat accggataat attttgaacc gcatggttca aaagtgaaag    60

acggtcttgc tgtcacttat agatggatcc gcgctgcatt agcta                   105


<210>  4
<211>  42
<212>  DNA
<213>  Artificial

<220>
<223>  Description of Artificial Sequence; Synthetic

<400>  4
cgtctttcac ttttgaacca tgcggttcaa aatattatcc gg                    42


<210>  5
<211>  63
<212>  DNA
<213>  Artificial

<220>
<223>  Description of Artificial Sequence; Synthetic

<400>  5
gatgtctcgg gatggcttcg gagttacgga tgtctcggga tgatctataa gtgacagcaa    60

gac                                                                   63


<210>  6
<211>  51
<212>  DNA
<213>  Artificial

<220>
<223>  Description of Artificial Sequence; Synthetic

<400>  6
cgtctttcac ttttgaacca taaaaaaaaa aaaaaaaaaa aaaaaaaaaa a             51


<210>  7
<211>  42
<212>  DNA
<213>  Artificial

<220>
<223>  Description of Artificial Sequence; Synthetic

<400>  7
catctctgct ggtccctcgg ctgcgtcggt tcgccataga cg                    42


<210>  8
<211>  42
<212>  DNA
<213>  Artificial

<220>
<223>  Description of Artificial Sequence; Synthetic

<400>  8
gcacattcac accgcgacgc agccgaggcc tgacctctat gc                    42


<210>  9
<211>  42
<212>  DNA
<213>  Artificial

<220>
<223>  Description of Artificial Sequence; Synthetic

<400>  9
gaccagcaga gatggcagcg acggcacccg tctatggcga ac                    42

<210>  10
<211>  42
<212>  DNA
<213>  Artificial

<220>
<223>  Description of Artificial Sequence; Synthetic

<400>  10
cggtgtgaat gtgcggtgcc gtcgctgcgc atagaggtca gg                    42


<210>  11
<211>  63
<212>  DNA
<213>  Artificial

<220>
<223>  Description of Artificial Sequence; Synthetic

<400>  11
catctctgct ggtccctcgg ctgcgtcggt tcgccataga cgatctataa gtgacagcaa    60

gac                                                               63


<210>  12
<211>  28
<212>  DNA
<213>  Artificial

<220>
<223>  Description of Artificial Sequence; Synthetic

<400>  12
gaccagcaga gatgcgtcta tggcgaac                                    28


<210>  13
<211>  28
<212>  DNA
<213>  Artificial

<220>
<223>  Description of Artificial Sequence; Synthetic

<400>  13
cggtgtgaat gtgcgcatag aggtcagg                                    28


<210>  14
<211>  52
<212>  DNA
<213>  Artificial

<220>
<223>  Description of Artificial Sequence; Synthetic

<400>  14
tttttttttt gatgtctcgg gatggcttcg gagttacgct ggcggtatca ac         52


<210>  15
<211>  51
<212>  DNA
<213>  Artificial

<220>
<223>  Description of Artificial Sequence; Synthetic

<400>  15
cccccccccc gatgtctcgg gatggcttcg gagttacgct ggcggtatca a                51


<210>  16
<211>  29
<212>  DNA
<213>  Artificial

<220>
<223>  Description of Artificial Sequence; Synthetic

<400>  16
cccccccccc atctataagt gacagcaag                                         29


<210>  17
<211>  21
<212>  DNA
<213>  Artificial

<220>
<223>  Description of Artificial Sequence; Synthetic

<400>  17
tttttttttt tttttttttt t                                                 21